# EUROPEAN PATENT APPLICATION

(11) **EP 4 588 908 A1**
(43) Date of publication of application: **23.07.2025**
(21) Application number: 24835413.6
(22) Date of filing: 04.07.2024
(51) Int. Cl.: C07C 59/72, A61K 31/192, A61K 31/215, A61P 9/12

(54) **NEW PROSTACYCLIN FOR TREATING PULMONARY HYPERTENSION**

(30) Priority: 05.07.2023 CN 202310819103
(71) Applicant: Shanghai Forefront Pharma Co., Ltd., Shanghai 201203 (CN)
(72) Inventor: REN, Yi, Shanghai 201203 (CN); ZHU, Lingui, Shanghai 201203 (CN); HUANG, Jincheng, Shanghai 201203 (CN)
(74) Representative: Santoro, Sofia
(86) International application number: PCT/CN2024/103697
(87) International publication number: WO 2025/007935

(57) **Abstract**

The present invention relates to a new prostacyclin for treating pulmonary hypertension. Specifically, the compound of the present invention has the structure as represented by formula (1), wherein the definition of each group and substituent is as described in the description. Compared with treprostinil and a salt thereof, the compound of the present invention has better right ventricular systolic pressure (RVSP) and a better Fulton index.

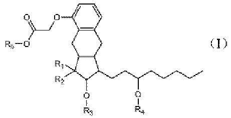

## Description

### FIELD OF THE INVENTION

The present invention relates to the field of pharmaceutical drugs, and specifically to a new prostacyclin for treating pulmonary hypertension.

### BACKGROUND OF THE INVENTION

Treprostinil is a medicament for the treatment of pulmonary artery hypertension (PAH). It is a prostacyclin derivative with the activity that can inhibit platelet aggregation and promote vasodilation. The approved dosage forms include injections, inhalations and oral tablets, wherein the drug substance of the injections and inhalations is treprostinil sodium salt.

However, the existing treprostinil sodium salt exhibits suboptimal efficacy in treating PAH. Therefore, there is an urgent need to develop more effective medicaments for PAH treatment.

Ralinepag is a medicament currently in Phase III clinical trials for the treatment of PAH. Its chemical structure is as follows:

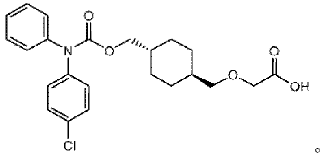

### SUMMARY OF THE INVENTION

The objective of the present invention is to provide a compound represented by formula (I), preparation methods thereof, and use thereof in treating PAH.

In a first aspect of the present invention, there is provided a compound represented by formula (I), or a solvate thereof, or a pharmaceutically acceptable salt thereof,

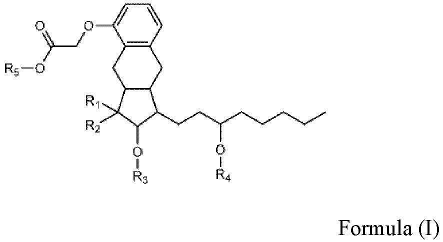

Wherein,
R₁ and R₂ are independently selected from the group consisting of hydrogen, deuterium, halogen, hydroxy, amino, substituted or unsubstituted C1-C6 alkyl, substituted or unsubstituted C2-C6 alkenyl, substituted or unsubstituted C2-C6 alkynyl, substituted or unsubstituted C1-C6 alkoxy, substituted or unsubstituted C3-C8 cycloalkyl, substituted or unsubstituted 4- to 8-membered heterocycloalkyl containing 1, 2 or 3 heteroatoms selected from N, O or S, substituted or unsubstituted C6-C10 aryl, substituted or unsubstituted 5- to 10-membered heteroaryl containing 1, 2 or 3 heteroatoms selected from N, O or S; 'substituted' refers to independent substitution by 1, 2 or 3 substituents selected from the group consisting of deuterium, halogen, hydroxyl, amino, C1-C6 alkyl, C2-C6 alkenyl, C2-C6 alkynyl, C1-C6 alkoxy, C3-C8 cycloalkyl, 4- to 8-membered heterocycloalkyl containing 1, 2 or 3 heteroatoms selected from N, O or S, R-substituted or unsubstituted C6-C10 aryl, R-substituted or unsubstituted 5-to 10-membered heteroaryl containing 1, 2 or 3 heteroatoms selected from N, O or S; R is selected from the group consisting of halogen, hydroxyl, C1-C6 alkyl, C1-C6 alkoxy;
or R₁, R₂ together with the C atoms to which they are attached form C3-C8 monocyclic cycloalkyl, C3-C8 bridged cycloalkyl, C7-C10 spirocycloalkyl, or 4- to 8-membered heterocycloalkyl containing 1, 2 or 3 heteroatoms selected from N, O or S;
and R₁ and R₂ are not simultaneously hydrogen;
R₃ and R₄ are independently selected from the group consisting of hydrogen, -(C=O)-C1-C6 alkyl, C1-C6 alkyl, halogenated C1-C6 alkyl, C3-C8 cycloalkyl, and halogenated C3-C8 cycloalkyl;
R₅ is selected from the group consisting of hydrogen, C1-C6 alkyl, halogenated C1-C6 alkyl, C3-C8 cycloalkyl, halogenated C3-C8 cycloalkyl, inorganic metal ion, and ammonium ion.

In another preferred embodiment, R₁ is hydrogen;
R₂ is selected from the group consisting of halogen, C1-C6 alkyl, halogenated C1-C6 alkyl, C1-C6 alkyl substituted by R-substituted or unsubstituted C6-C10 aryl, hydroxy-substituted C1-C6 alkyl, C2-C6 alkenyl, C3-C8 cycloalkyl, 4- to 8-membered heterocycloalkyl containing 1, 2 or 3 heteroatoms selected from N, O or S, C6-C10 aryl, and 5-to 10-membered heteroaryl containing 1, 2 or 3 heteroatoms selected from N, O or S;
R is selected from the group consisting of halogen, C1-C6 alkyl, and C1-C6 alkoxy.

In another preferred embodiment, R₁ is hydrogen;
R₂ is C1-C6 alkyl.

In another preferred embodiment, R₁ is hydrogen;
R₂ is selected from the group consisting of methyl, ethyl, propyl, isopropyl, butyl, isobutyl, tert-butyl, neopentyl, and tert-pentyl.

In another preferred embodiment, R₁ is hydrogen;
R₂ is ethyl.

In another preferred embodiment, R₁ is H, halogen, methyl, or ethyl;
R₂ is selected from the group consisting of halogen, methyl, ethyl, propyl, isopropyl, butyl, isobutyl, tert-butyl, neopentyl, tert-pentyl, trifluoromethyl, difluoromethyl, monofluoromethyl, monofluoroethyl, trifluoroethyl, difluoroisopropyl, hydroxy-substituted ethyl, vinyl, propenyl, ethynyl, propynyl, benzyl, diphenylmethyl, methoxybenzyl, monochlorobenzyl, phenyl-substituted ethyl, and hydroxy-substituted benzyl.

In another preferred embodiment, R₁, R₂ together with the C atoms to which they are attached form a group selected from the group consisting of cyclopropyl, cyclobutyl, cyclopentyl and cyclohexyl.

In another preferred embodiment, R₃ and R₄ are hydrogen.

In another preferred embodiment, R₅ is hydrogen.

In another preferred embodiment, the inorganic metal ion is selected from the group consisting of Li⁺, Na⁺, K⁺, Rb⁺, Cs⁺, Mg²⁺, Ca²⁺, Ba²⁺, Zn²⁺, and Al³⁺.

In another preferred embodiment, R₅ is Na⁺.

In another preferred embodiment, the ammonium ion is an ammonium ion corresponding to a base selected from the group consisting of ammonia, methylamine, ethylamine, dimethylamine, trimethylamine, diethylamine, triethylamine, ethanolamine, diethanolamine, triethanolamine, tert-butylamine, tromethamine, meglumine, morpholine, piperazine, piperidine, pyridine, ethylenediamine, N,N'-dibenzylethylenediamine, proline, phenylalanine, aspartic acid, glutamic acid, and lysine.

In another preferred embodiment, the ammonium ion is a cation.

In another preferred embodiment, the pharmaceutically acceptable salt is an alkali metal salt or an alkaline earth metal salt.

In another preferred embodiment, the pharmaceutically acceptable salt is a salt of the compound selected from the group consisting of lithium salt, sodium salt, potassium salt, cesium salt, magnesium salt, calcium salt, barium salt, zinc salt, and aluminum salt.

In another preferred embodiment, the pharmaceutically acceptable salt is a salt of the compound selected from the group consisting of lithium salt, sodium salt, potassium salt, and cesium salt.

In another preferred embodiment, the pharmaceutically acceptable salt is a salt of the compound selected from the group consisting of magnesium salt, calcium salt, barium salt, zinc salt, and aluminum salt.

In another preferred embodiment, the pharmaceutically acceptable salt is a sodium salt of the compound.

In another preferred embodiment, the pharmaceutically acceptable salt is a salt formed by the compound and a nitrogen-containing organic base.

In another preferred embodiment, the pharmaceutically acceptable salt is a salt formed by the compound and a base selected from the group consisting of ammonia, methylamine, ethylamine, dimethylamine, trimethylamine, diethylamine, triethylamine, ethanolamine, diethanolamine, triethanolamine, tert-butylamine, tromethamine, meglumine, morpholine, piperazine, piperidine, pyridine, ethylenediamine, N,N'-dibenzylethylenediamine, proline, phenylalanine, aspartic acid, glutamic acid, and lysine.

In another preferred embodiment, the solvate is a hydrate.

In another preferred embodiment, the compound has a structure represented by formula (II) or formula (III):

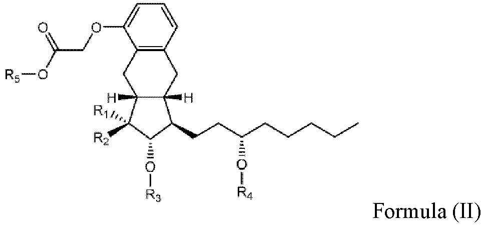

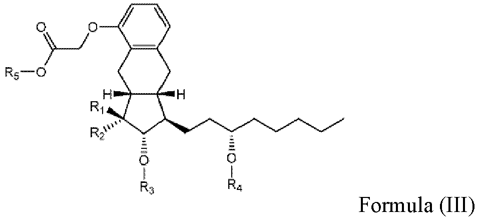

Wherein, R₁, R₂, R₃, R₄, and R₅ are as defined above.

In another preferred embodiment, the compound is selected from the group consisting of the following:

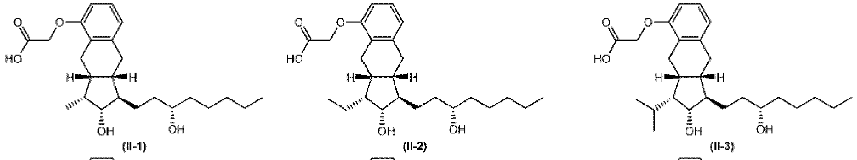

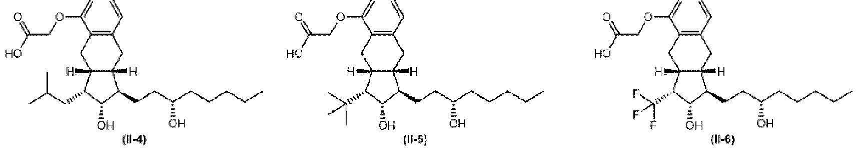

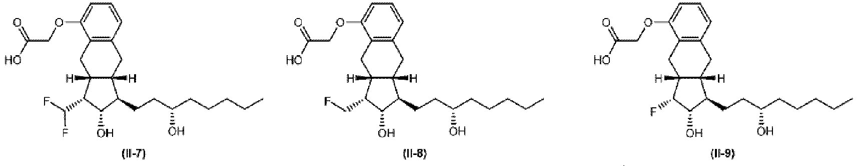

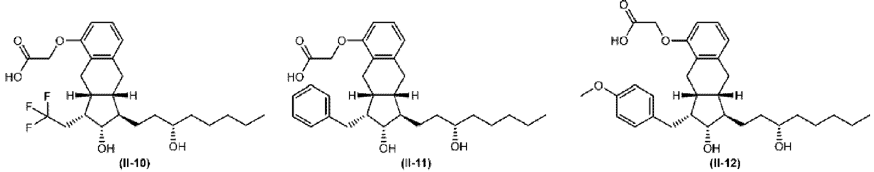

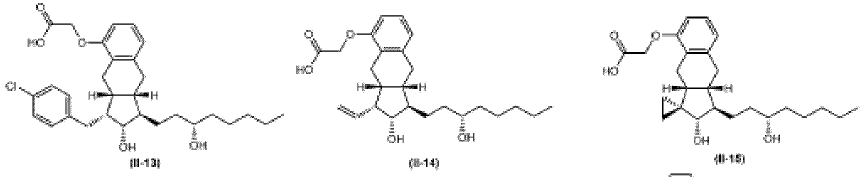

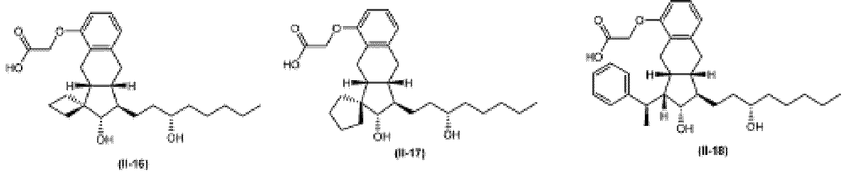

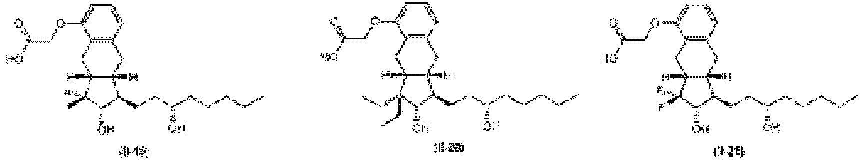

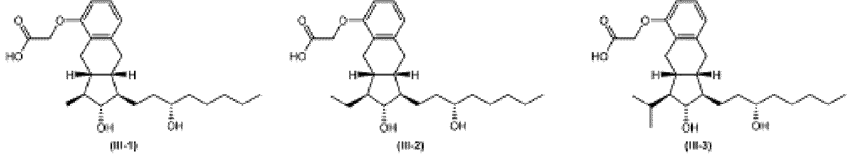

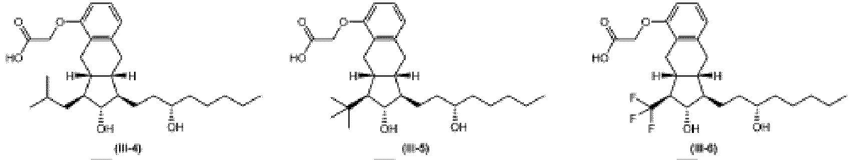

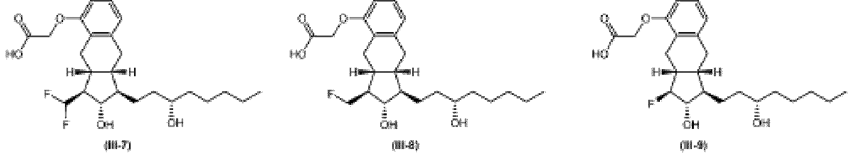

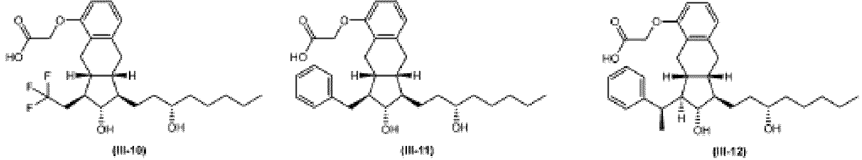

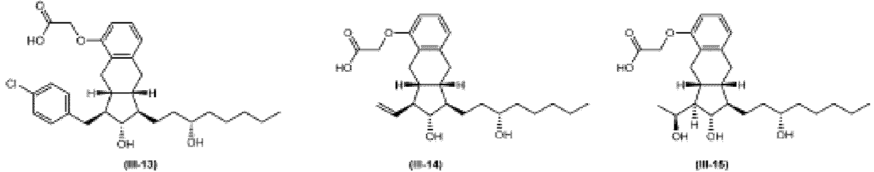

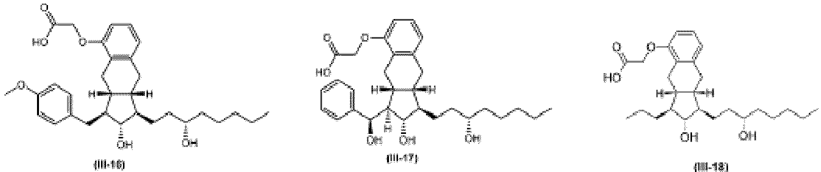

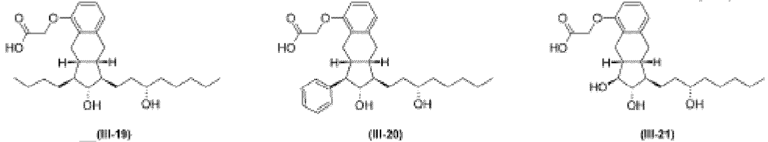

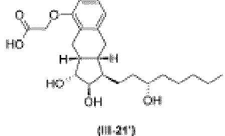

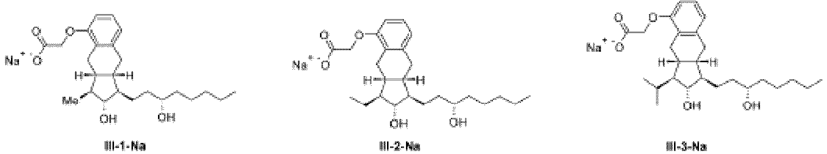

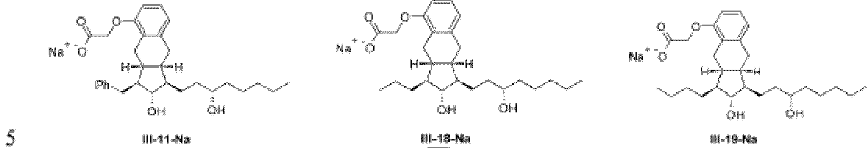

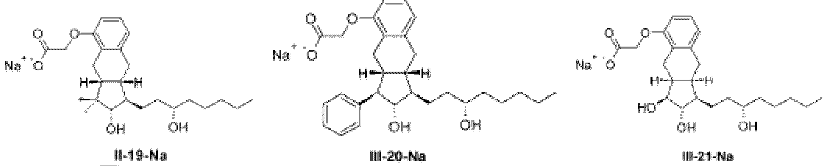

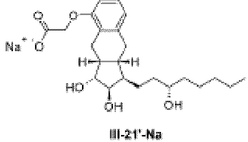

In a second aspect of the present invention, there is provided a pharmaceutical composition comprising pharmaceutically acceptable carriers and a safe and effective amount of one or more compounds as described in the first aspect of the present invention, or a solvate thereof, or a pharmaceutically acceptable salt thereof.

In a third aspect of the present invention, there is provided a use of the compound as described in the first aspect of the present invention, or a solvate thereof, or a pharmaceutically acceptable salt thereof, in the manufacture of a medicament for treating PAH.

In another preferred embodiment, the medicament is in an oral formulation.

In another preferred embodiment, the dose of the medicament is 5-7 mg/kg/day, preferably 6 mg/kg/day.

In another preferred embodiment, the medicament is used for a purpose selected from the group consisting of the following:
1) Reducing right ventricular systolic pressure (RVSP);
2) Reducing Fulton index;
3) Reducing pulmonary vascular resistance (PVR);
4) Treating pulmonary hypertension caused by interstitial lung disease;
5) Treating pulmonary hypertension caused by COPD;
6) Treating idiopathic pulmonary fibrosis.

It should be understood that within the scope of the present invention, the above-mentioned technical features of the present invention and the technical features specifically described therewith below (such as embodiments) can be combined with each other to form a new or preferred technical scheme. Due to limited space, it will not be elaborated here.

### BRIEF DESCRIPTION OF THE DRAWINGS

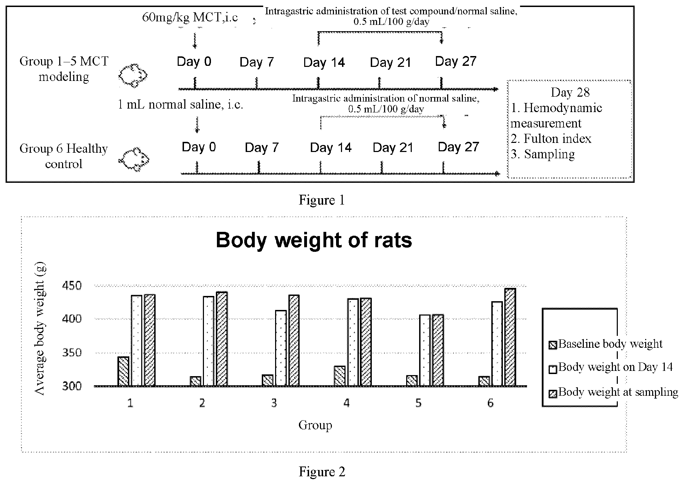

Figure 1 is a representation of the technical route of the present invention.
Figure 2 is a bar graph corresponding to the body weight data in Table 1 obtained in the present invention.

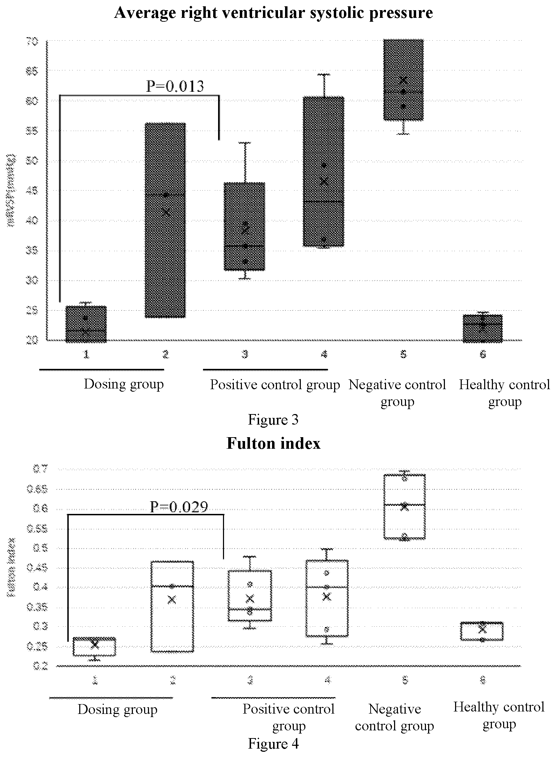

Figure 3 is a bar graph corresponding to the RVSP data in Table 2 obtained in the present invention.
Figure 4 is a bar graph corresponding to the Fulton index data in Table 3 obtained in the present invention.

### DETAILED DESCRIPTION OF THE INVENTION

After long-term and in-depth research, by optimizing the structure of the existing treprostinil sodium salt, the inventors have developed a new prostacyclin with enhanced therapeutic efficacy in treating PAH. Specifically, by substitution at the R₁ and R₂ positions of the compound of formula I (preferably with C1-C6 alkyl, or more preferably with mono-C1-C6 alkyl), a compound of formula (I) having better RVSP and a better Fulton index is obtained. On this basis, the inventors have established the present invention.

### Definitions

In the present invention, unless otherwise specified, all terms used have their usual meaning in the art, which will be clear to the skilled person.

In the present invention, the term "halogen" refers to F, Cl, Br or I.

In the present invention, "C1-C6 alkyl" refers to linear or branched alkyl group having 1 to 6 carbon atoms, such as methyl, ethyl, propyl, isopropyl, butyl, isobutyl, tert-butyl, neopentyl, tert-pentyl, or the like.

In the present invention, the term "C2-C6 alkenyl" refers to linear or branched alkenyl group having 2 to 6 carbon atoms and containing one double bond, including but not limited to ethenyl, propenyl, butenyl, isobutenyl, pentenyl, hexenyl and the like.

In the present invention, the term "C2-C6 alkynyl" refers to linear or branched alkynyl group having 2 to 6 carbon atoms and containing a triple bond, including but not limited to ethynyl, propynyl, butynyl, isobutynyl, pentynyl, hexynyl and the like.

In the present invention, the term "C3-C8 cycloalkyl" refers to cyclic alkyl group having 3 to 8 carbon atoms in the ring, including but not limited to cyclopropyl, cyclobutyl, cyclopentyl, cyclohexyl, cycloheptyl, cyclooctyl and the like.

In the present invention, the term "C1-C6 alkoxy" refers to linear or branched alkoxy group having 1 to 6 carbon atoms, including but not limited to methoxy, ethoxy, propoxy, isopropoxy, butoxy and the like. Preferably, the alkoxy group is C1-C4 alkoxy.

In the present invention, the term "heterocycloalkyl" refers to 4- to 8-membered heterocycloalkyl group containing 1, 2 or 3 heteroatoms selected from N, O or S, including (but not limited to) the following groups:

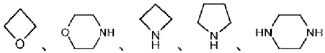

In the present invention, the term "aromatic ring" has the same meaning as "aryl", preferably "C6-C10 aryl". The term "C6-C10 aryl" refers to aromatic ring group having 6 to 10 carbon atoms and containing no heteroatoms in the ring, such as phenyl, naphthyl, and the like.

In the present invention, the term "aromatic heterocycle" has the same meaning as "heteroaryl", referring to heteroaryl group containing one to multiple heteroatoms. For example, "5- to 10-membered heteroaryl" refers to aromatic heterocycle containing 1 to 4 heteroatoms selected from oxygen, sulfur and nitrogen, and 3 to 10 carbon atoms. Examples include but are not limited to furanyl, thienyl, pyridyl, pyrazolyl, pyrrolyl, N-alkylpyrrolyl, pyrimidinyl, pyrazinyl, imidazolyl, tetrazolyl, and the like. The heteroaryl ring can be fused to aryl, heterocyclyl or cycloalkyl ring, wherein the ring connected to the parent structure is the heteroaryl ring. The heteroaryl can be optionally substituted or unsubstituted.

In the present invention, the term "halogenated" refers to substitution by halogen.

In the present invention, the term "substituted" refers to the replacement of one or multiple hydrogen atoms on a specific group with specific substituent(s). Specific substituents are the substituents described hereinabove or that appearing in the embodiments. Unless otherwise specified, a substituted group can have a substituent selected from the specified group at any substitutable position of the group, and the substituents can be the same or different at each position. It should be understood by ones skilled in the art that combinations of substituents contemplated in the present invention are those combinations that are stable or chemically achievable. The substituents
include but are not limited to halogen, hydroxyl, carboxyl (-COOH), C1-C6 alkyl, C2-C6 alkenyl, C2-C6 alkynyl, C3-C8 cycloalkyl, 3- to 12-membered heterocycloalkyl, aryl, heteroaryl, C1-C8 aldehyde group, C2-C10 acyl group, C2-C10 ester group, amino group, C1-C6 alkoxy, C1-C10 sulfonyl, and the like.

In the present invention, the term "1-6" refers to 1, 2, 3, 4, 5 or 6. Other similar terms independently have similar meanings. The term "multiple" refers to 2-6, such as 2, 3, 4, 5 or 6.

The term "ester group" refers to a -C(O)-O-R' or R'-C(O)-O- structure, wherein R independently represents hydrogen, C1-C6 alkyl, C3-C6 cycloalkyl, C6-C10 aryl, heteroaryl, heterocycloalkyl, as defined above.

It should be understood that when a group is present simultaneously at multiple different positions of a compound, its definition at each position is independent of each other and can be the same or different. That is, the term "selected from the group consisting of' has the same meaning as the term "independently selected from the group consisting of".

The term "COPD" refers to chronic obstructive pulmonary disease.

The Fulton index reflects the organ remodeling caused by PAH. The change in this index indicates that the new drug has the potential to reverse the altered cardiopulmonary structure caused by PAH, which means it can not only alleviate the symptoms of PAH (symptomatic treatment), but also reverse or even cure PAH (definitive treatment). This is a goal that current pharmaceutical drugs for treating PAH cannot achieve, but one that new drugs for PAH aim to reach.

### Compound

The present invention provides a compound represented by formula I, or a solvate thereof, or a pharmaceutically acceptable salt thereof, 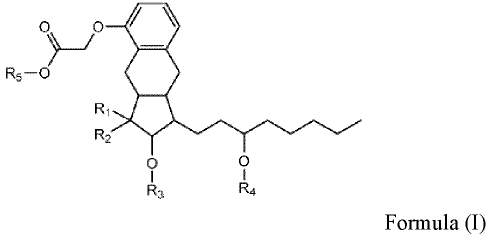 wherein, each group is as defined above.

In another preferred embodiment, any R₁, R₂, R₃, R₄ and R₅ in the compound is independently a corresponding group in the specific compound of the present invention.

As used herein, the term "pharmaceutically acceptable salt" refers to a salt formed by the compound of the present invention and an acid or base, which is suitable for use as a pharmaceutical. Pharmaceutically acceptable salt includes inorganic salts and organic salts. One preferred class of salts those formed by the compound of the present invention and an acid. Suitable acids for salt formation include but are not limited to amino acids such as proline, phenylalanine, aspartic acid, glutamic acid, lysine, and the like.

Another preferred class of salts is those formed by the compound of the present invention and a base, such alkali metal salts (e.g., sodium salt or potassium salt), alkaline earth metal salts (e.g., magnesium salt or calcium salt), ammonium salts (e.g., lower alkanolammonium salt), and other pharmaceutically acceptable amine salts (e.g., methylamine salt, ethylamine salt, propylamine salt, dimethylamine salt, trimethylamine salt, diethylamine salt, triethylamine salt, tert-butylamine salt, ethylenediamine salt, hydroxyethylamine salt, dihydroxyethylamine salt, trihydroxyethylamine salt, and amine salts formed by morpholine, piperazine, and lysine, respectively).

The term "solvate" refers to a complex formed by coordination of the compound of the present invention with solvent molecules at a specific ratio. "Hydrate" refers to a complex formed by coordination of the compound of the present invention with water.

The present invention also includes co-crystals formed by the compound of the present invention and compatible compounds.

In addition, the compounds of the present invention also include prodrugs of the compounds represented by formula (I). The term "prodrug" includes a compound which can be biologically active or inactive, and which, when administered by an appropriate route, undergoes metabolism or chemical reaction *in vivo* to convert into the compound of formula (I), or a salt or solution of the compound of formula (I). The prodrugs include (but are not limited to) carboxylate, carbonate, phosphate, nitrate, sulfate, sulfone, sulfoxide, amide, carbamate, azo compound, phosphoramide, glucoside, ether, acetal of the compounds, and the like.

It should be understood that the embodiments of the present invention more describe the preparation methods of the compound of formula (I) in the present invention in more detail, but these specific methods do not limit the present invention in any way. The compound of the present invention can also be readily prepared by optionally combining various synthetic methods described herein or known in the art. Such a combination of methods can be easily performed by ones skilled in the art to which the present invention belongs.

Typically, the raw materials and reagents used in the process for preparing the compound of the present invention are commercially available unless otherwise specified.

### Pharmaceutical Compositions and Routes of Administration

The present invention also provides a pharmaceutical composition comprising pharmaceutically acceptable carriers and a safe and effective amount of one or more compounds, or a solvate thereof, or a pharmaceutically acceptable salt thereof.

The pharmaceutical composition of the present invention comprises a safe and effective amount of the compound of the present invention or a pharmacologically acceptable salt thereof and pharmacologically acceptable excipients or carriers. Wherein, the "safe and effective amount" refers to the amount of the compound that is sufficient to significantly improve the condition without causing serious side effects. Typically, the pharmaceutical composition comprises 1-2000 mg of the compound of the present invention per unit dose, more preferably 10-1000 mg of the compound of the present invention per unit dose. Preferably, the "unit dose" is a capsule or tablet, and can also be an injection, an inhalation, or an oral formulation.

"Pharmaceutically acceptable carrier" refers to one or more compatible solid or liquid fillers or gel substances, which are suitable for human use and must be sufficiently pure and sufficiently low in toxicity. "Compatible" herein means that the components in the composition can be blended with the compound of the present invention and with each other without significantly reducing the efficacy of the compound. Examples of pharmaceutically acceptable carriers include cellulose and its derivatives (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifying agents (such as Tween^{®}), moisturizing agents (such as sodium lauryl sulfate), colorants, flavoring agents, stabilizing agents, antioxidants, preservatives, pyrogen-free water, etc.

The pharmaceutical composition is in the dosage form of injections, capsules, tablets, pills, powders or granules. There is no particular limitation on the administration routes of the compound or pharmaceutical composition of the present invention. Representative routes of administration include (but are not limited to) oral, rectal, parenteral (intravenous, intramuscular or subcutaneous), oral-nasal inhalation, anal, vaginal and topical administration.

Solid dosage forms for oral administration include capsules, tablets, pills, powders and granules. In these solid dosage forms, the active compound is mixed with at least one commonly-used inert excipient such as lubricant, e.g., talc, calcium stearate, magnesium stearate, solid polyethylene glycol, sodium lauryl sulfate, or mixtures thereof. In the case of capsules, tablets and pills, the composition can also comprise buffering agents.

Solid dosage forms such as tablets, sugar pills, capsules, pills and granules can be prepared using coatings and capsule shell materials such as enteric coatings and other materials well known in the art. They can contain opacifying agents, and the release of the active compound or compounds in such compositions can be delayed in a certain portion of the digestive tract. Examples of embedding components which can be used are polymeric substances and waxy substances. If necessary, the active compound can also be in microencapsulated form with one or more of the above-mentioned excipients.

Liquid dosage forms for oral administration include pharmaceutically acceptable emulsions, solutions, suspensions, syrups, or tinctures. In addition to the active compound, the liquid dosage form can contain inert diluent commonly used in the art, such as water or other solvents, solubilizing agents and emulsifying agents, e.g., ethanol, isopropanol, ethyl carbonate, ethyl acetate, propylene glycol, 1,3-butanediol, dimethylformamide and oils, in particular cottonseed oil, peanut oil, corn germ oil, olive oil, castor oil and sesame oil or mixtures thereof.

Besides these inert diluents, the composition can also include adjuvants such as moisturizing agents, emulsifying and suspending agents, sweeteners, flavoring agents, and fragrances.

In addition to the active compound, the suspension can contain suspending agents such as ethoxylated isostearyl alcohol, polyoxyethylene sorbitol and sorbitan ester, microcrystalline cellulose, aluminum methanolate and agar, or mixtures thereof, and the like.

The composition for parenteral injection can include physiologically acceptable sterile aqueous or anhydrous solutions, dispersions, suspensions or emulsions, and sterile powders for reconstitution into sterile injectable solutions or dispersions. Suitable aqueous and non-aqueous carriers, diluents, solvents or excipients include water, ethanol, polyols and suitable mixtures thereof.

Dosage forms of the compound of the present invention for topical administration include ointments, powders, patches, sprays and inhalations.

The active ingredient is mixed under sterile conditions with physiologically acceptable carriers and any preservatives, buffers, or propellants if necessary.

The compound of the present invention can be administered alone or in combination with other pharmaceutically acceptable compounds.

The treatment of the present invention can be used alone or in combination with other treatments or therapeutic agents.

When using the pharmaceutical composition, a safe and effective amount of the compound of the present invention is administered to mammals (such as humans) in need of treatment, wherein the dose for administration is a pharmaceutically effective dose. For a person weighing 60 kg, the daily dose is usually 1-2000 mg, preferably 50-1000 mg. Certainly, the specific dose should also take into account factors such as the route of administration and the patient's health status, all of which fall within the expertise of a skilled physician.

Compared with the prior art, the present invention has the following main advantages:
(1) The compound of the present invention has better therapeutic effects on PAH;
(2) The compound of the present invention has better RVSP and a better Fulton index;
(3) Compared with Ralinepag (United Therapeutics Corporation) which is in Phase III clinical trials, the corresponding animal experiment data of the compound in the present invention are also better than those of Ralinepag;
(4) Based on body weight data and clinical observations, the feeding, defecation, activity, and paw strength of the rats in the dosing group were no different from those in the healthy control group.

The present invention is further described below with reference to specific embodiments. It should be understood that these embodiments are only used to illustrate the present invention and are not used to limit the scope of the present invention. In the following embodiments, experimental methods without specific conditions are generally performed according to common conditions as described in Molecular Cloning: A Laboratory Manual (Sambrook et al., New York: Cold Spring Harbor Laboratory Press, 1989), or according to the conditions recommended by the manufacturer. Unless otherwise specified, percentages and portions are calculated by weight.

Unless otherwise defined, all professional and scientific terms used herein have the same meanings as commonly understood by ones skilled in the art. In addition, any methods and materials similar or equivalent to those described herein can be applied to the methods of the present invention. The preferred embodiments and materials described herein are for exemplary purposes only.

### EXAMPLES

II-N or III-N and its corresponding sodium salt II-N-Na or III-N-Na are prepared according to the following procedures:

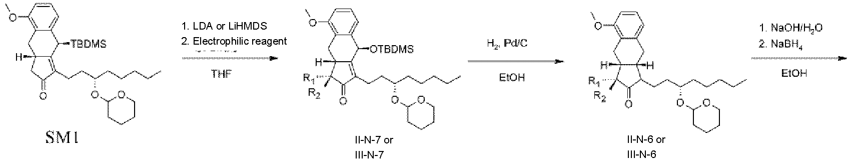

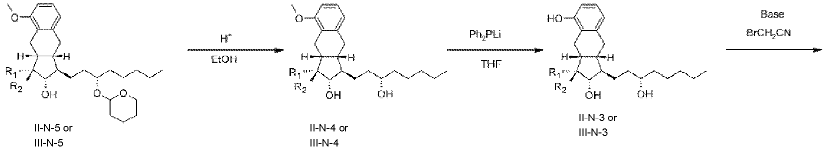

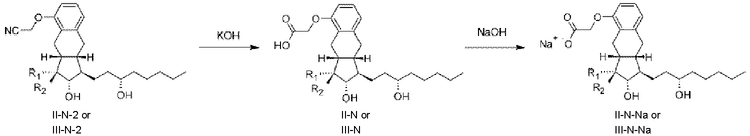

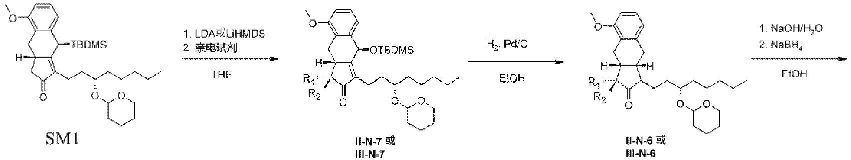

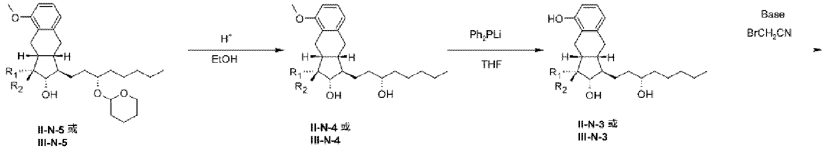

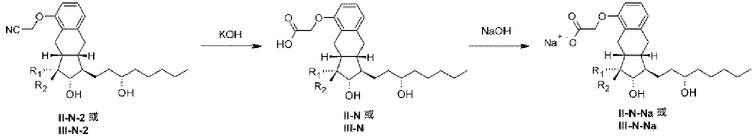

Note: II-N represents compounds such as II-1, II-2, etc.; III-N represents compounds such as III-1, III-2, etc. Step 1:
To a reaction flask, the starting material (SM1) and tetrahydrofuran were added and then cooled to -65°C. LDA or LiHMDS (1.0-5.0 equivalents) was added dropwise at this temperature. After the addition was completed, the mixture was stirred at this temperature for 3 hours. To the reaction system, the electrophilic reagent (1.0-5.0 equivalents) was then added. After the addition was completed, the mixture was allowed to slowly warm up and stirred overnight. To the reaction system, a saturated aqueous ammonium chloride solution was added to quench the reaction. The mixture was extracted, washed, dried, concentrated, and purified by column chromatography to obtain product II-N-7 or III-N-7. The product was characterized by LC-MS and ¹H NMR, respectively.

Wherein, the electrophile was selected from the group consisting of the following:

| Product | Electrophilic reagent |
|---|---|
| III-1 or III-1-Na | Methyl iodide |
| III-2 or III-2-Na | Ethyl iodide |
| III-3 or III-3-Na | 2-Iodopropane |
| III-11 or III-11-Na | Benzyl bromide |
| III-18 or III-18-Na | 1 -Iodopropane |
| III-19 or III-19-Na | 1-Iodobutane |
| II-19 or II-19-Na | Methyl iodide |
| III-21 or III-21-Na and III-21' or III-21'-Na | (1R)-(-)-(10-Camphorsulfonyl)oxaziridine |

### Step 2:

II-N-7 was dissolved in anhydrous ethanol. The mixture was transferred to a hydrogenation reactor, and palladium on carbon was added to hydrogenate overnight. The palladium on carbon was removed by filtration, and the resulting filtrate was concentrated under reduced pressure. The crude product was purified by column chromatography to obtain the product II-N-6 or III-N-6. The product was characterized by LC-MS and ¹H-NMR, respectively.

### Step 3:

II-N-6 was dissolved in anhydrous ethanol and then cooled to -25°C--15°C. An appropriate amount of 2M sodium hydroxide aqueous solution was added. The mixture was stirred at this temperature for 2-4 hours, sodium borohydride (1.0-2.0 equivalents) was added while maintaining the temperature, and stirring was continued for 4-6 hours. The mixture was extracted, washed, dried, concentrated, and purified by column chromatography to obtain product II-N-5 or III-N-5. The product was characterized by LC-MS and ¹H-NMR, respectively.

### Step 4:

II-N-5 or III-N-5 was dissolved in anhydrous ethanol, and 1M dilute hydrochloric acid was added. The mixture was stirred at room temperature for 2-6 hours. The mixture was extracted, washed, dried, concentrated, and purified by column chromatography to obtain product II-N-4 or III-N-4. The product was characterized by LC-MS and ¹H-NMR, respectively.

### Step 5:

Diphenylphosphine (5.0-10.0 equivalents) was dissolved in anhydrous tetrahydrofuran and cooled to -25°C--15°C. N-butyl lithium (5.0-10.0 equivalents) was added dropwise, and after the addition was completed, stirring was continued at this temperature for 2-4 hours to obtain a solution of lithium diphenylphosphine in tetrahydrofuran. To another reaction flask, II-N-4 or III-N-4 was added and dissolved in tetrahydrofuran. The freshly prepared solution of lithium diphenylphosphide in tetrahydrofuran was slowly added to the mixture at room temperature. After the addition was completed, the reaction system was refluxed overnight. Heating was stopped, and the mixture was cooled in an ice-water bath. The reaction was quenched with saturated ammonium chloride solution. The mixture was extracted, washed, dried, filtered, concentrated under reduced pressure, and purified by column chromatography to obtain product II-N-3 or III-N-3. The product was characterized by LC-MS and ¹H-NMR, respectively.

### Step 6:

Dissolve II-N-3 or III-N-3 was dissolved in acetone, and potassium carbonate and bromoacetonitrile (1.5-3.0 equivalents each) were added in sequence. The mixture was stirred at room temperature overnight. Insoluble solid was removed by filtration, and the filtrate was directly concentrated under reduced pressure. The crude product was purified by column chromatography to obtain product II-N-2 or III-N-2. The product was characterized by LC-MS and ¹H-NMR, respectively.

### Step 7:

II-N-2 or III-N-2 was dissolved in tetrahydrofuran, and a 20% potassium hydroxide aqueous solution was added. The mixture was stirred until hydrolysis was complete. The pH was adjusted to 3-4 with 2M hydrochloric acid. The mixture was extracted, washed, dried, filtered, and concentrated under reduced pressure to obtain the crude product, and then purified by column chromatography to obtain product II-N or III-N. The product was characterized by LC-MS and ¹H-NMR, respectively.

### Step 8:

II-N or III-N was dissolved in tetrahydrofuran, and an equivalent amount of solid sodium hydroxide was added. The mixture was stirred overnight. After concentration under reduced pressure, the mixture was beaten to slurry with acetonitrile to obtain a white solid precipitate. The precipitate was filtered, washed, and dried to yield sodium salt II-N-Na or III-N-Na. The product was characterized by LC-MS and ¹H-NMR, respectively.

The characterization data of the compounds prepared by the above methods of the present invention are shown in Table A, Table B and Table C.

**Table A**

| Compound structure and number | Characterization data |
|---|---|
| 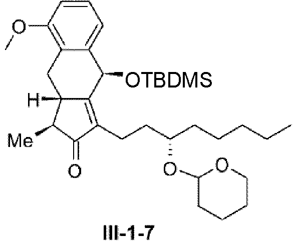 | ¹H NMR (400 MHz, d⁶-DMSO)δ7.29-7.24(m, IH),6.95-6.90(m,2H),[5.60(s)+5.53(s), IH],4.49-4.48(m, IH),3.78(m,3H),3.73-3.69(m, IH),3.49-3.26(m,2H),2.83-2.77(m, IH),2.32-2.10(m,4H),1.71-1.11(m,20H),0.87-0.78(m,12H),0.13-0.06(m,6H). |
| | LRMS: Calcd. for C₃₄H₅₄O₅Si [M + Na]⁺:593.4, found 593.3. |
| 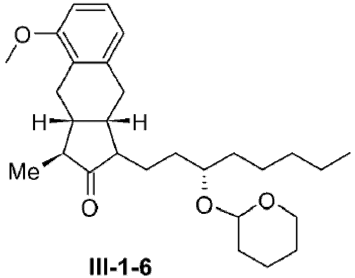 | ¹H NMR (400 MHz, d⁶-DMSO)δ7.21-7.13(m, IH),6.86-6.77(m,2H),4.57-4.52(m, IH),4.07-3.80(m,2H),3.77-3.68(m,4H),3.54-3.43(m,2H),3.39-3.32(m, IH),2.46-2.37(m, IH),2.31-2.13(m,2H),2.11-1.99(m,2H),1.77-1.13(m,16H),1.14-1.08(m,5H),0.89-0.79(m,3H). |
| | LRMS: Calcd. for C₂₈H₄₂O₄[M + Na]⁺:465.3, found 465.3. |
| 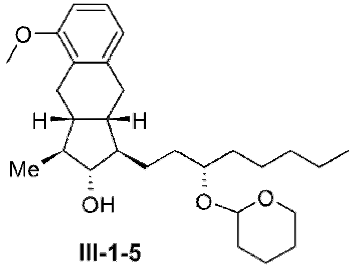 | ¹H NMR (400 MHz, d⁶-DMSO)δ7.08-7.04(m, IH),6.81-6.72(m,2H),4.64-4.61(m, IH),4.45(t, J=6 Hz, IH),3.86-3.68(m,4H),3.58-3.38(m,2H),2.94-2.87(m, IH),2.68-2.56(m,2H),2.48-2.29(m,2H),1.80-1.18(m,19H),1.11(s, IH),1.06-0.93(m,5H),0.89-0.83(m,3H). |
| | LRMS: Calcd. for C₂₈H₄₄O₄[M + Na]⁺:467.3.found 467.3. |
| 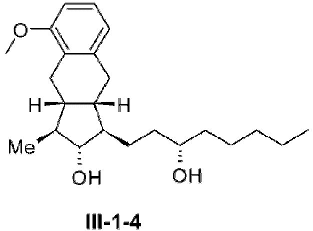 | ¹H NMR (400 MHz, d⁶-DMSO)δ7.08-7.04(m,1H),6.80(d, J=8 Hz,1H),6.73(d, J=8Hz,1H),4.41(d,J=6.3 Hz,1H),4.21(d,7=5.3 Hz,1H),3.75(s,3H),2.94-2.88(m,1H),2.68-2.57(m,2H),2.43(dd, J=14.8 Hz, J=5.6Hz,1H),2.36(dd, J =14.0 Hz,7=6.0 Hz,1H),1.81-1.71(m,1H),1.69-1.59(m,2H),1.51-1.16(m,12H),1.10-0.92(m,5H),0.87(t, J=6.8 Hz,3H). LRMS: |
| | Calcd. for C₂₃H₃₆O₃[M +Na]⁺:380.3, found 380.3. |
| 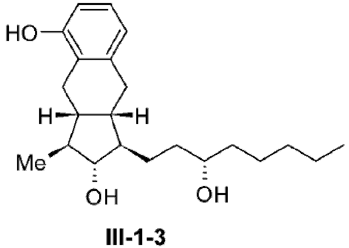 | ¹H NMR (400 MHz, d⁶-DMSO)δ9.02(s,1H),6.89-6.85(m,1H),6.64-6.62(m,1H),6.58-6.56(m,1H),4.41(d,J=6.0 Hz,1H),4.21(d, J=5.2Hz,1H),2.93-2.87(m,1H),2.63-2.53(m,2H),2.42(dd, J=14.4 Hz,7=5.6 Hz,1H),2.32(dd, J=14.4 Hz,7=6.0 Hz,1H),1.81-1.70(m,1H),1.69-1.58(m,2H),1.52-1.20(m,12H),1.09-0.92(m,5H),0.87(t, J=6.8 Hz,3H). |
| | LRMS: Calcd. for C₂₂H₃₄O₃[M +Na]⁺:369.3, found 369.3. |
| 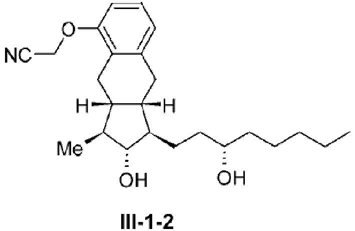 | ¹H NMR (400 MHz, d⁶-DMSO)δ7.14(t, J =8.0 Hz, IH),6.96(d,7=8.0 Hz, IH),6.88(d,7=7.2 Hz, IH),5.14(s,2H),4.47(d, J=6.4 Hz, IH),4.23(d, J=5.2 Hz, IH),2.94-2.88(m, IH),2.69-2.61(m,2H),2.48-2.37(m,2H),1.84-1.73(m, IH),1.72-1.59(m,2H),1.53-1.15(m,12H),1.09-0.91(m,5H),0.87(t, J=6.8 Hz,3H). |
| | LRMS: Calcd. for C₂₄H₃₅NO₃[M + Na]⁺:408.3, found 408.3. |
| 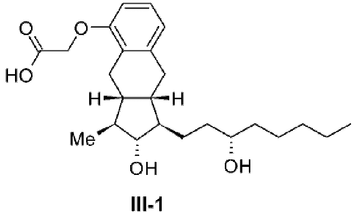 | ¹H NMR (400 MHz, d⁶-DMSO)δ12.88(br s, IH),7.03(3 J=8.0 Hz, IH),6.76(d, J =8.0 Hz, IH),6.68(d,7=8.0 Hz, IH),4.63(s,2H),4.45(d,7=6.4 Hz, IH),4.21(d, J=5.6 Hz, IH),3.35-3.33(m, IH),2.95-2.89(m, IH),2.72-2.62(m,2H),2.50-2.33(m,2H),1.81-1.71(m, IH),1.71-1.59(m,2H),1.52-1.15(m,12H),1.11-1.00(m,3H),0.96(d,7=6.4 Hz,3H),0.87(t,J=6.8 Hz,3H). |
| | LRMS: Calcd. for C₂₄H₃₆O₅[M + Na]⁺:427.3, found 427.2. |
| 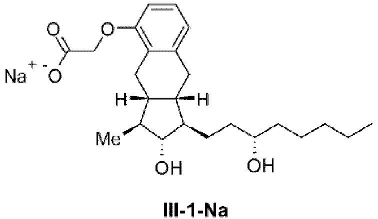 | ¹H NMR (400 MHz, D₂O)δ7.09(t, J=8.0 Hz,1H),6.77(d,7=8.0 Hz,1H),6.75(d,7=8.0 Hz,1H),4.42(s,2H),3.56(m,1H),3.10(t, J=9.6 Hz,1H),2.85(dd, J=14.4 Hz,J=5.6 Hz,1H),2.65(dd, J=14.4 Hz, J=5.6Hz,1H),2.46(dd, J=14.4 Hz, J=5.6 Hz,1H),2.37(dd, J=14.4 Hz, J =5.6 Hz,1H),1.73-1.19(m,18H),1.01(d, J =6.4 Hz,3H),0.90(t, J=6.4 Hz,3H). |
| | LRMS: Calcd. for C₂₄H₃₅NaO₅[M + H]⁺:427.3, found 427.2. |
| 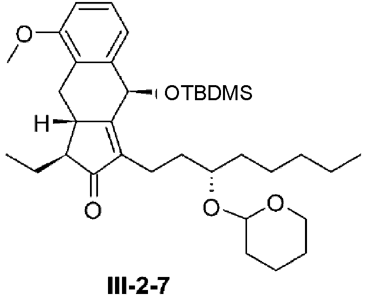 | ¹H NMR (400 MHz, CDCI₃)δ7.25-7.20(m,1H),6.91(t,7=6.8 Hz,1H),6.78(dd, J=8.0 Hz, J =4.8 Hz,1H),[5.59(s)+5.48(s),1H],4.57-4.47(m,1H),3.91-3.92(m,4H),3.57-3.45(m,2H),3.43-3.32(m,1H),3.08-2.98(m,1H),2.51-2.13(m,4H),2.11-2.00(m,1H),1.93-1.75(m,2H),1.73-1.15(m,15H),1.03(t, J =7.2 Hz,3H),0.93-0.78(m,12H),0.17-0.06(m,6H). |
| | LRMS: Calcd. for C₃₅Hs₅₆O₅Si [M + Na]⁺:607.4, found 607.3o |
| 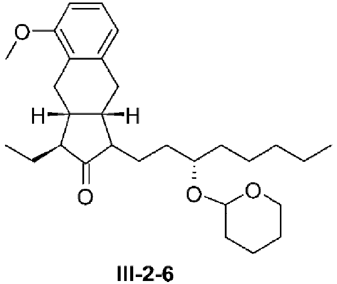 | ¹H NMR (400 MHz, d⁶-DMSO)δ7.14-7.04(m,1H),6.84-6.64(m,2H),4.67-4.52(m,1H),3.83-3.68(m,4H),3.62-3.36(m,2H),3.13-2.28(m,5H),2.26-1.87(m,3H),1.83-1.15(m,20H),0.94-0.80(m,6H) |
| | LRMS: Calcd. for C₂₉H₄₄O₄[M + Na]⁺:479.3, found 479.3. |
| 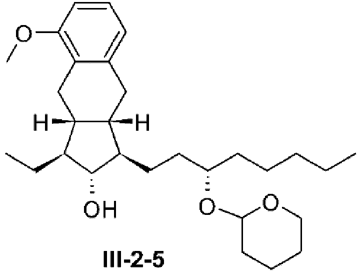 | ¹H NMR (400 MHz,d⁶-DMSO)δ7.06(t, J =8.0 Hz, IH),6.80(d,7=8.0 Hz, IH),6.78(d, J=8.0Hz, IH),4.66-4.58(m, IH),4.41(t,7=6.8 Hz, IH),3.88-3.68(m,4H),3.59-3.48(m, IH),3.47-3.38(m, IH),3.07-2.99(m, IH),2.72-2.59(m,2H),2.48-2.27(m,2H),1.84-1.68(m,3H),1.67-1.18(m,21H),0.93(t,J=6.8 Hz,3H),0.89-0.81(m,3H). LRMS: Calcd. for C₂₉H₄₆O₄[M + Na]⁺:481.3, found 481.3. |
| 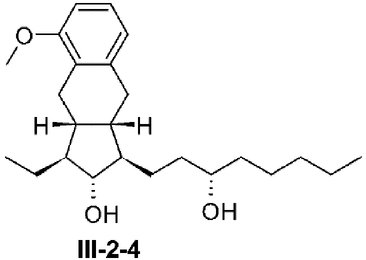 | ¹H NMR (400 MHz, d⁶-DMSO)δ7.06(t, J =8.0 Hz,1H),6.80(d,7=8.0 Hz,1H),6.78(d, J=8.0 Hz,1H),4.40(d, J=6.8 Hz,1H),4.21(d,7=5.2 Hz,1H),3.75(s,3H),3.38-3.30(m,1H),3.09-2.99(m,1H),2.69-2.60(m,2H),2.48-2.31(m,2H),1.79-1.68(m,2H),1.65-1.16(m,14H), 1.11-0.98(m,2H),0.93(t, J=6.8 Hz,3H),0.87(t, J =7.2 Hz,3H). |
| | LRMS: Calcd. for C₂₄H₃₃O₃[M + Na]+:397.3, found 397.2. |
| 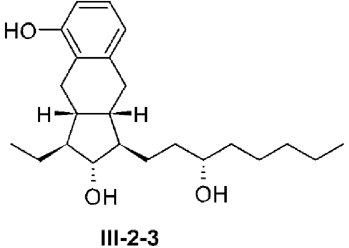 | ¹H NMR(400MHz,d⁶-DMSO)δ9.02(s,1H),6.87(t,7=8.0 Hz,1H),6.63(d, J=8.0Hz,1H),6.57(d,7=8.0 Hz,1H),3.38-3.28(m,1H),3.06-2.99(m,1H),2.68-2.56(m,2H),2.40(dd,7=7.2 Hz,7=4.8 Hz,1H),2.18(dd,7=7.2 Hz,J=4.8 Hz,1H),2.48-2.31(m,2H),1.78-1.67(m,2H),1.65-1.16(m,14H),1.11-0.98(m,2H),0.94(t, J=7.2Hz,3H),0.87(t, J=6.4Hz,3H). |
| | LRMS: Calcd. for C₂₃H₃₆O₃[M + Na]+:383.3, found 383.2. |
| 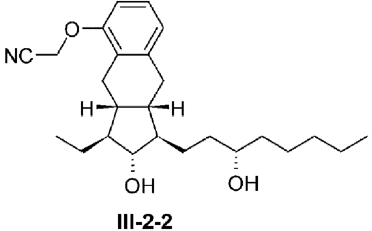 | ¹H NMR (400 MHz, CDCl₃)δ7.14(t,7=8.0 Hz,1H),6.90(d,7=8.4 Hz,1H),6.82(d,7=8.4 Hz,1H),4.76(s,2H),3.65-3.57(m,1H),3.34(t,J=9.4 Hz,1H),3.10-2.66(m,3H),2.53(dd, J=7.2Hz, J=5.2Hz,1H),2.45(dd,7=7.2 Hz,7=5.6 Hz,1H),1.95-1.81(m,2H),1.74-1.14(m,17H),1.03(t, J=7.6 Hz,3H),0.90(t, J=6.8 Hz,3H). |
| | LRMS: Calcd. for C2₅H₃₇NO₃[M +Na]⁺:422.3, found 422.4. |
| 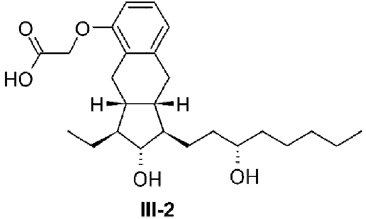 | ¹H NMR (400 MHz, d⁶-DMSO)δ12.93(br s,1H),7.03(3 J=8.0 Hz,1H),6.75(d, J=8.4 Hz,1H),6.68(d,7=8.4 Hz,1H),4.62(s,2H),4.41(d,7=6.4 Hz,1H),4.21(s,1H),3.08-3.00(m,1H),2.78-2.62(m,2H),2.48-2.30(m,2H),1.81-1.69(m,2H),1.67-1.00(m,17H),0.94(t, J=7.2 Hz,3H),0.87(t, J=6.4 Hz,3H) |
| | LRMS: Calcd. for C₂₄H₃₃O₅[M + Na]⁺:441.3, found 441.3. |
| 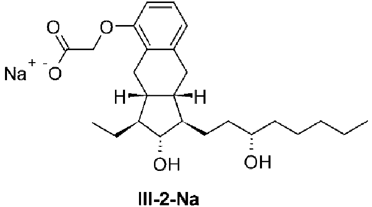 | ¹H NMR (400 MHz, d⁶-DMSO)δ6.93(t, J =8.0 Hz,1H),6.63(d,7=7.2 Hz,1H),6.75(d,7=8.0 Hz,1H),4.40(s,2H),4.21(s,1H),4.05(s,2H),3.40-3.33(m,1H),3.06(t, J =9.6 Hz,1H),2.81(dd, J =14.4 Hz, J=5.2Hz,1H),2.66(dd, J=13.6 Hz, J =5.6 Hz,1H),2.39-2.26(m,2H),1.73-1.06(m,18H),0.94(t,7=7.6 Hz,3H),0.87(t, J=6.4 Hz,3H) |
| | LRMS: Calcd. for C₂₅H₃₇NaO₅[M + H]⁺:441.3, found 441.3. |
| 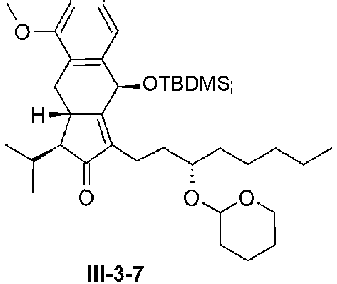 | ¹H NMR (400 MHz, d⁶-DMSO)δ7.29-7.25(m,1H),6.94-6.90(m,2H),[5.59(s)+5.52(s),1H],4.50-4.41(m,1H),3.78(s,3H),3.74-3.65(m,1H),3.42-3.37(m,1H),3.05-2.99(m,1H),2.19-2.11(m,3H),1.74-1.67(m,1H),1.60-1.38(m,9H),1.27-1.08(m,10H),1.02(d,3H),0.84-0.81(m,3H),0.80-0.78(s,9H),0.71(d,3H),0.14-0.10(m,6H). |
| | LCMS: Calculated for C₃₆H₅₆O₅Si [M + Na]⁺:621.3,found 621.30 |
| 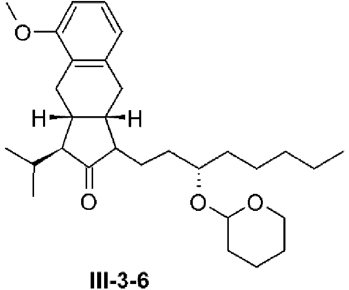 | The product III-3-6 crude was used in the next step directly. |
| | LC-MS characterization confirmed it as the target product. |
| | LCMS: Calculated for C₃₆H₅₆O₅Si [M + Na]⁺:493.3, found 493.3. |
| 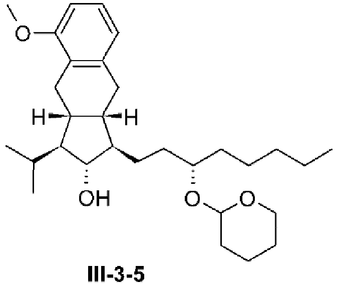 | ¹H NMR (400 MHz,d⁶-DMSO)δ7.06(t, J =8.0, IH),6.80(d,7=8.0 Hz, IH),6.73(d, J=8.0 Hz, IH),4.64-4.61(m, IH),4.36-4.33(t, J=7.6Hz, IH),3.83-3.77(m, IH),3.75(s,3H),3.54-3.52(m, IH),3.44-3.40(m, IH),3.21-3.15(m, IH),2.71-2.65(m,2H),2.40-2.31(m,2H),1.82-1.56(m,6H),1.54-1.39(m,8H),1.29-1.24(m,7H),1.12-1.07(m,2H),0.97-0.94(m,3H),0.90-0.82(m,6H). |
| | LCMS: Calculated for C₃₀H₄₈O₄[M + Na]⁺:495.3, found 495.3. |
| 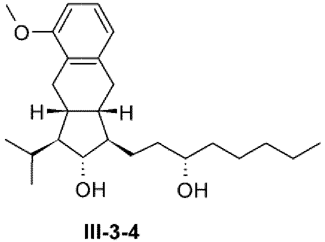 | ¹H NMR (400 MHz, d⁶-DMSO)δ7.06(t, J =8.0 Hz,1H),6.81-6.79(d,7=8.4 Hz,1H),6.74-6.72(d, J=7.6 Hz,1H),4.33(d, J =6.8 Hz,1H),4.20(t,J=5.2 Hz,1H),3.75(s,3H),3.35-3.34(m,1H),3.22-3.15(m,1H),2.72-2.65(m,2H),2.39-2.32(m,2H),1.82-1.57(m,4H),1.47-1.24(m,11H),1.14-1.07(m,2H),0.98-0.95(m,3H),0.91-0.85(m,6H). |
| | LCMS: Calculated for C₂₅H₄₀O₃[M + Na]⁺:411.3, found 411.3. |
| 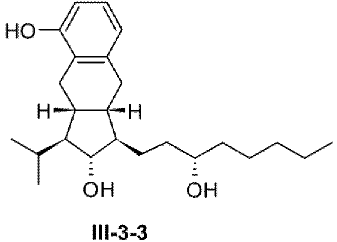 | ¹H NMR (400 MHz,d⁶-DMSO)δ9.02(s,1H),6.87(t,7=7.6 Hz,1H),6.63(d,7=7.6 Hz,1H),6.57-6.56(d,7=7.2 Hz,1H),4.32-4.31(m,1H),4.20-4.19(m,1H),3.19-3.18(m,1H),2.71-2.61(m,2H),2.33-2.28(m,2H),1.82-1.58(m,4H),1.46-1.24(m,12H),1.17-1.10(m,2H),0.98-0.96(m,3H),0.92-0.85(m,6H). |
| | LCMS: Calculated for C₂₄H₃₃O₃[M +Na]⁺:397.2, found 397.2. |
| 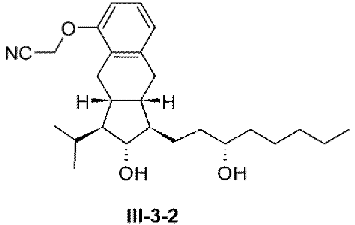 | ¹H NMR (400 MHz,^6-DMSO)(57,14(t, J =8.0 Hz,1H),6.96-6.94(d,7=8.4 Hz,1H),6.89-6.87(d,7=7.6 Hz,1H),5.14(s,2H),4.39-4.23(m,2H),3.19-3.18(m,1H),2.73-2.66(m,2H),2.43-2.35(m,2H),1.84-1.69(m,3H),1.60-1.56(m,1H),1.49-1.25(m,12H),1.15-1.06(m,2H),0.98-0.96(m,3H),0.91-0.85(m,6H)O |
| | LCMS: Calculated for C₂₆H₃₉NO₃[M + Na]⁺:436.3, found 436.3. |
| 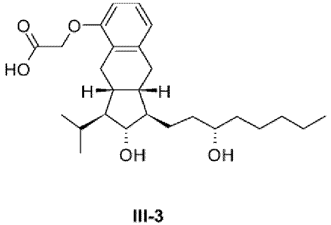 | ¹H NMR(400MHz,d⁶-DMSO)δ6.94(t, J =8.0 Hz,1H),6.65(d,/=7.2 Hz,1H),6.60-6.58(d,7=8.4 Hz,1H),4.34-4.33(m,1H),4.21-4.20(m,1H),4.15(s,2H),3.24-3.19(m,1H),2.87-2.82(m,1H),2.71-2.66(m,1H),2.34-2.26(m,2H),2.01-1.99(m,1H),1.82-1.61(m,4H),1.47-1.19(m,14H),0.98-0.96(m,3H),0.93-0.91(m,3H),0.88-0.85(m,3H). |
| | LCMS: Calculated for C₂₆H₄₀O₅[M + Na]⁺:455.3, found 455.3. |
| 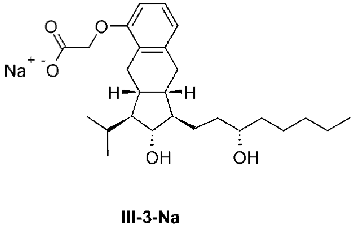 | ¹H NMR (400 MHz,d⁶-D₂O)δ7.10-7.08(m,1H),6.83-6.82(m,1H),6.70-6.68(m,1H),4.38(s,2H),3.59-3.40(m,2H),2.80-2.71(m,2H),2.47-2.39(m,2H),1.89-1.79(m,3H),1.57-1.22(m,14H),0.94-0.79(m,9H). LCMS: Calculated for C₂₆H₃₉NaO₅[M + H]⁺:455.3, found 455.3. |
| 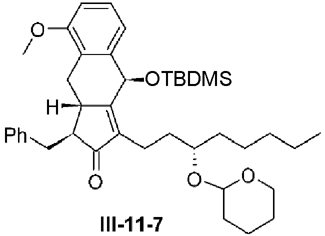 | ¹H NMR(400MHz, d⁶-DMSO)δ7.77(d, J =8.8 Hz,2H),7.54-7.44(m,3H),7.31-7.27(m,1H),6.99-6.87(m,2H),[5.72(s)+5.65(s),1H],4.50-4.25(m,2H),3.71-3.65(m,5H),3.43-3.25(m,2H),3.07(m,2H),2.47-2.27(m,2H),2.00-1.90(m,1H),1.77-1.07(m,17H),0.84-0.78(m,12H),(0.18(s)+0.16(s),3H),0.11(s,3H). |
| | LRMS: Calcd. for C₄₀H₅₈O₅Si[M + Na]⁺:667.4, found 667.3. |
| 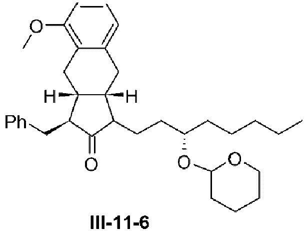 | ¹H NMR (400 MHz, d⁶-DMSO)δ 7.31-7.25(m,2H),7.22-7.03(m,4H),6.77-6.64(m,2H),4.65-4.52(m, IH),3.82-3.67(m,4H),3.55-3.36(m,2H),2.93-2.80(m,2H),2.69-2.53(m,2H),2.34-1.95(m,5H),1.74-1.23(m,19H),0.88-0.84(m,3H). |
| | LRMS: Calcd. for C₃₄H₄₆O₄[M + Na]⁺:541.3, found 541.3. |
| 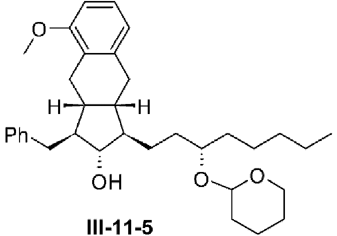 | ¹H NMR (400 MHz, d⁶-DMSO)δ7.31-7.27(m,2H),7.22-7.17(m,3H),7.03(dd,7=7.6 Hz, J=8.0 Hz, IH),6.74(d, J=8.4 Hz, IH),6.67(d, J=8.4 Hz, IH),4.63-4.60(m, IH),4.57(t,7=6.4 Hz, IH),3.84-3.75(m, IH),3.64(s,3H),3.55-3.48(m, IH),3.45-3.48(m, IH),3.07(m, IH),2.81(dd, J=13.2 Hz, J=4.0 Hz, IH),2.57(dd, J=14 Hz, J=6.0 Hz, IH),2.52-2.45(m, IH),2.37-2.29(m, IH),2.24-2.12(m,2H),2.03-1.18(m,21H),1.06-1.12(m, IH),0.88-0.84(m,3H) |
| | LRMS: Calcd. for C₃₄H₄₈O₄[M + Na]+:543.4, found 543.3. |
| 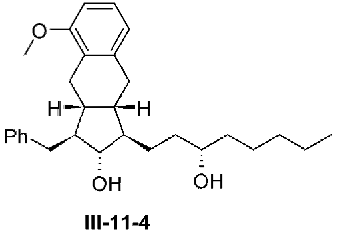 | ¹H NMR (400 MHz, d⁶-DMSO)δ7.31-7.27(m,2H),7.22-7.15(m,3H),7.02(dd, J =7.6 Hz,7=8.4 Hz,1H),6.74(d,7=8.0 Hz,1H),6.67(d,7=8.0 Hz,1H),4.56(d, J=6.4 Hz,1H),4.21(d,7=5.2 Hz,1H),3.64(s,3H),3.35-3.32(m,1H),3.08-3.00(m,1H),2.92(dd, J=13.2 Hz,7=4.0 Hz,1H),2.57(dd, J=13.2 Hz,7=4.0 Hz,1H),2.49-2.45(m,1H),2.33(dd, J=13.2 Hz,7=5.6 Hz,1H),2.24-2.11(m,2H),1.83-1.64(m,2H),1.64-1.54(m,1H),1.47-1.19(m,12H),1.07-0.98(m,1H),0.87(t, J=6.8Hz,3H)O LRMS: Calcd. for C₂₉H₄₀O₃[M + Na]⁺:459.3, found 459.3. |
| 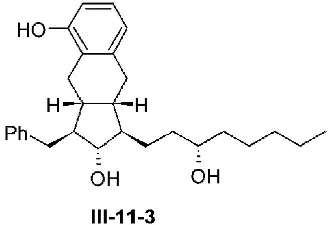 | ¹H NMR(400MHz,(d⁶-DMSO)δ8.92(s,1H),7.29-7.25(m,2H),7.21-7.15(m,3H),6.85(dd, J=7.6 Hz, J=7.6 Hz,1H),6.59(d, J=8.0 Hz,1H),6.54(d, J=7.2 Hz,1H),4.52(d, J=6.4Hz,1H),4.19(d, J=5.2Hz,1H),3.37-3.32(m,1H),3.08-3.00(m,1H),2.85(dd, J=13.2 Hz,7=4.4 Hz,1H),2.62-2.53(m,2H),2.40(dd, J=14.4 Hz,7=6.4 Hz,1H),2.28(dd, J=14.0 Hz,J=6.4 Hz,1H),2.19(dd, J=14.4 Hz, J=6.0 Hz,1H),1.76-1.66(m,1H),1.65-1.52(m,2H),1.48-1.15(m,12H),1.12-1.02(m,1H),0.86(t, J=6.8 Hz,3H). |
| | LRMS: Calcd. for C₂₈H₃₈O₃[M + Na]⁺:445.3, found 445.2. |
| 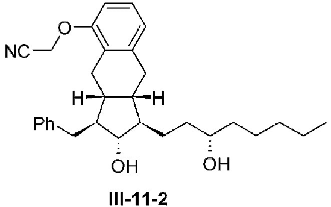 | ¹H NMR (400 MHz,d⁶-DMSO)δ7.31(t, J =7.2 Hz,2H),7.23-7.18(m,3H),7.10(dd, J=8.0Hz, J=7.6 Hz, IH),6.89(d, J=8.4 Hz, IH),6.83(d, J=7.6Hz, IH),4.98(s,2H),4.62(d,7=6.4 Hz, IH),4.23(d, J =5.6 Hz, IH),3.40-3.2(m,1H),3.09-3.00(m, IH),2.96(dd, J=13.2 Hz, J=4.0Hz, IH),2.60(dd, J=10.0 Hz,7=6.0 Hz, IH),2.49-2.42(m,2H),2.35(dd, J=14.0 Hz,7=5.6 Hz, IH),2.17(dd, J=14.4 Hz, J=5.6Hz, IH),2.05(dd, J=14.4 Hz,7=5.2 Hz, IH),1.84-1.68(m,2H),1.63-1.55(m, IH),1.47-1.23(m,12H),1.07-1.00(m, IH),0.86(1, J=6.8 Hz,3H). |
| | LRMS: Calcd. for C₃₀H₃₀NO₃[M + Na]⁺:484.3, found 484.2. |
| 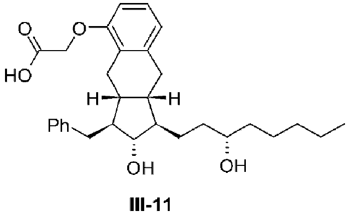 | ¹H NMR (400 MHz, d⁶-DMSO)δ7.27(t, J =7.2 Hz,2H),7.21-7.14(m,3H),6.93(dd,7=8.0 Hz,7=7.6 Hz,1H),6.64(d,7=7.2 Hz,1H),6.56(d,7=7.6 Hz,1H),4.54(br s,1H),4.28-4.10(m,3H),3.49-3.37(m,1H),3.07-2.98(m,1H),2.87(dd,/=13.2 Hz, J=4,4 Hz,1H),2.61-2.52(m,2H),2.43-2.20(m,3H),1.81-1.70(m,1H),1.69-1.52(m,2H),1.50-1.16(m,12H),1.11-1.02(m,1H),0.86(t, J=6.8 Hz,3H). |
| | LRMS: Calcd. for C₃₀H₄₀O₅[M + Na]⁺:503.3, found 503.4. |
| 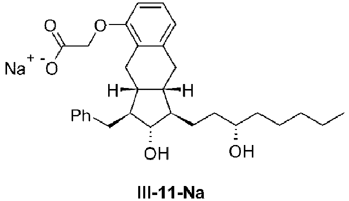 | ¹H NMR (400 MHz, D₂O)δ7,39-7.29(m,5H),7.10(t, J=8.0 Hz,1H),6.80(d, J =7.2 Hz,1H),6.72(d,7=8.4 Hz,1H),4.33(s,2H),3.65-3.55(m,1H),3.27(t,7=7.2 Hz,1H),3.06-2.95(m,1H),2.72-2.17(m,6H),1.92-1.82(m,1H),1.79-1.72(m,1H),1.68-1.17(m,15H),0.87(t, J=6.0Hz,3H). LRMS: Calcd. for C₃₀H₃₉NaO₅[M + H]⁺:503.3, found 503.3. |
| 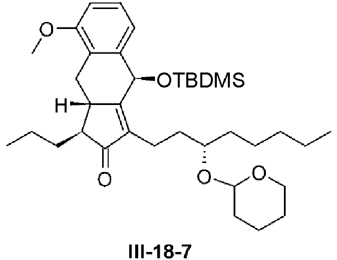 | ¹H NMR (400 MHz, d⁶-DMSO)δ7.29-7.24(m, IH),6.94-6.90(m,2H),[5.60(s)+5.52(s), IH],4.51-4.37(m, IH),3.77(s,3H),3.75-3.65(m, IH),3.45-3.35(m,2H),3.33-3.24(m, IH),3.08(s, IH),2.95-2.87(m, IH),2.36-2.10(m,4H),1.77-1.07(m,19H),0.90(1, J =1.2 Hz,3H),0.85-0.76(m,12H),0.13-0.07(m,6H) |
| | LRMS: Calcd. for C₃₆H₅₈O₅Si [M + Na]⁺:621.4, found 621.3. |
| 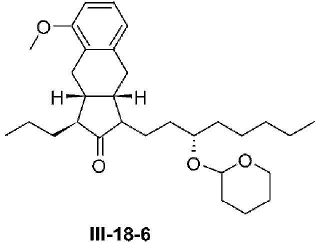 | ¹H NMR (400 MHz,d⁶-DMSO)δ7.11-7.05(m, IH),6.82-6.65(m,2H),4.66-4.53(m, IH),3.82-3.69(m,4H),3.60-3.47(m, IH),3.45-3.37(m, IH),3.12-2.73(m,2H),2.71-2.23(m,3H),2.19-2.08(m,3H),1.81-1.16(m,23H),0.96-0.80(m,6H). |
| | LRMS: Calcd. for C₃₀H₄₆O₄[M + Na]⁺:493.3, found 493.4. |
| 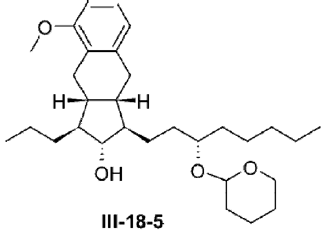 | ¹H NMR (400 MHz, d⁶-DMSO)δ7.06(dd, J =8.0 Hz, J=7.6 Hz,1H),6.80(d, J=8.0 Hz,1H),6.72(d,7=7.2 Hz,1H),4.64-4.60(m,1H),4.40(t,J=6.8 Hz,1H),3.86-3.76(m,1H),3.73(s,3H),3.54-3.51(m,1H),3.46-3.38(m,1H),3.05-2.98(m,1H),2.68-2.59(m,2H),2.49-2.30(m,2H),1.79-1.68(m,3H),1.66-1.20(m,22H),1.11-1.00(m,1H),0.93-0.82(m,6H). |
| | LRMS: Calcd. for C₃₀H₄₈O₄[M + Na]⁺:495.4, found 495.3. |
| 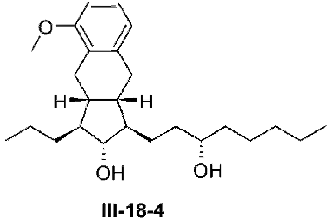 | ¹H NMR(400MHz, d⁶-DMSO)δ7.06(dd, J =8.0 Hz, J=7.6 Hz,1H),6.80(d, J=7.6 Hz,1H),6.73(d,7=7.2 Hz,1H),4.40(d, J=6.8 Hz,1H),4.21(d,7=5.6 Hz,1H),3.74(s,3H),3.38-3.33(m,1H),3.05-2.97(m,1H),2.67-2.60(m,2H),2.45(dd, J =13.2 Hz,7=5.2 Hz,1H),2.35(dd, J=13.2 Hz, J=5.2 Hz,1H),1.79-1.67(m,2H),1.65-1.56(m,1H),1.53-1.16(m,15H),1.12-1.01(m,2H),0.88(t,7=6.8 Hz,3H),0.87(t, J=6.8 Hz,3H). |
| | LRMS: Calcd. for C₂₅H₄₀O₃[M + Na]⁺:411.3, found 411.3. |
| 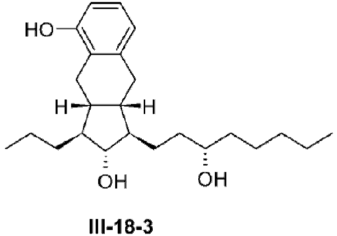 | ¹H NMR(400MHz, d⁶-DMSO)δ9.05(s,1H),6.87(t,J=7.6 Hz,1H),6.63(d, J=8.0Hz,1H),6.56(d,J=7.2 Hz,1H),4.40(d, J =4.8 Hz,1H),4.36(t, J=4.8Hz,1H),4.26(d, J=4.8Hz,1H),3.48-3.40(m,1H),3.06-2.96(m,1H),2.67-2.56(m,2H),2.40(dd, J =14.4 Hz, J =4.8 Hz,1H),2.31(dd, J =14.4 Hz,7=4.8 Hz,1H),1.78-1.55(m,3H),1.53-1.16(m,15H),1.11-1.03(m,4H),0.93-0.82(m,6H)o |
| | LRMS: Calcd. for C₂₄H₃₈O₃[M + Na]⁺:397.3, found 397.3. |
| 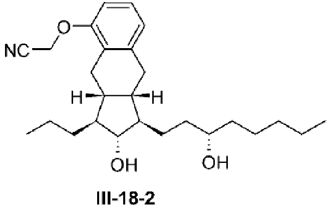 | ¹H NMR(400MHz, d⁶-DMSO)δ7.14(t, J =8.0 Hz,1H),6.95(d,7=8.4 Hz,1H),6.87(d,7=7.2 Hz,1H),5.14(s,2H),4.43(d, J=6.8 Hz,1H),4.23(d, J=5.2 Hz,1H),3.38-3.30(m,1H),3.06-2.97(m,1H),2.70-2.60(m,1H),2.48-2.35(m,2H),1.83-1.71(m,2H),1.66-1.56(m,1H),1.52-1.19(m,15H),1.09-1.00(m,2H),0.88(t,J=6.8 Hz,3H),0.87(t, J=6.8 Hz,3H). |
| | LRMS: Calcd. for C₂₄H₃₈NO₃[M + Na]⁺:397.3, found 397.3. |
| 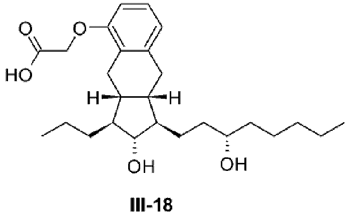 | ¹H NMR (400 MHz, d⁶-DMSO)δ6.95(t, J =7.6 Hz,1H),6.66(d,7=7.6 Hz,1H),6.60(d,7=8.4 Hz,1H),4.52-4.13(m,2H)4.24(s,2H),3.68-3.21(brs,1H),3.40-3.30(m,1H),3.03(t,J=9.6 Hz,1H),2.85-2.74(m,1H),2.70-2.59(m,1H),2.44-2.25(m,2H),1.74-1.56(m,3H),1.54-1.06(m,17H),0.94(t,7=7.6 Hz,3H),0.87(t,7=6.4 Hz,3H) |
| | LRMS: Calcd. for C₂₆H₄₀O₅[M + Na]⁺:455.3, found 455.3. |
| 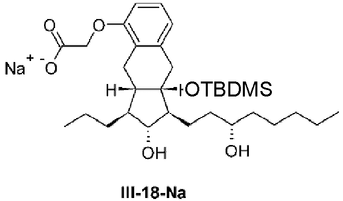 | ¹H NMR (400 MHz, D₂O)δ7.17(t, J=8.0 Hz, IH),6.89(d, J=7.2Hz, IH),6.78(d, J=8.4Hz, IH),4.46(s,2H),3.71-3.62(m, IH),3.29(t,7=9.6 Hz, IH),2.89-2.71(m,2H),2.65-2.42(m,2H),1.95-1.80(m,2H),1.73-1.17(m,18H),0.99-0.85(m,6H). |
| | LRMS: Calcd. for C₂₆H₃₉NaO₅[M + H]⁺:455.3, found 455.3. |
| 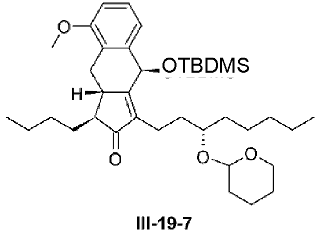 | ¹H NMR (400 MHz, d⁶-DMSO)δ7.29-7.24(m, IH),6.95-6.90(m,2H),(5.60(s)+5.52(s), IH),4.51-4.37(m, IH),3.78(s,3H),3.75-3.65(m, IH),3.45-3.35(m,2H),3.33-3.24(m, IH),2.95-2.87(m, IH),2.35-2.08(m,4H),1.76-1.63(m,2H),1.63-1.06(m,21H),0.88-0.74(m,14H),0.13-0.07(m,6H). |
| | LRMS: Calcd. for C₃₇H₆₀O₅Si[M + Na]⁺:635.4, found 635.4. |
| 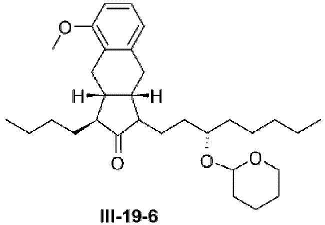 | ¹H NMR (400 MHz, d⁶-DMSO)δ7.11-7.05(m, IH),6.82-6.66(m,2H),4.66-4.53(m, IH),3.83-3.68(m,4H),3.60-3.37(m,2H),3.12-2.72(m,2H),2.71-2.23(m,3H),2.20-2.08(m,2H),2.02-1.87(m, IH),1.83-1.14(m,24H),0.96-0.80(m,6H). |
| | LRMS: Calcd. for C₃₁H₄₈O₄[M + Na]+:507.4, found 507.3. |
| 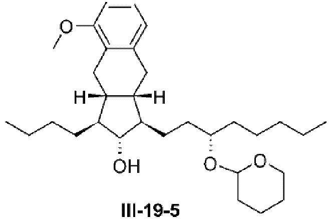 | ¹H NMR (400 MHz, d⁶-DMSO)δ7.06(dd, J =8.0 Hz, J=7.6 Hz, IH),6.80(d, J=8.0 Hz, IH),6.72(d,7=7.2 Hz, IH),4.64-4.60(m, IH),4.40(t, J=6.8Hz, IH),3.86-3.76(m, IH),3.74(s,3H),3.57-3.49(m, IH),3.46-3.39(m, IH),3.05-2.96(m, IH),2.68-2.57(m,2H),2.49-2.30(m,2H),1.80-1.68(m,3H),1.66-1.19(m,23H),1.11-0.97(m,2H),0.93-0.82(m,6H). |
| | LRMS: Calcd. for C₃₁H₅₀O₄[M + Na]⁺:509.4, found 509.3. |
| 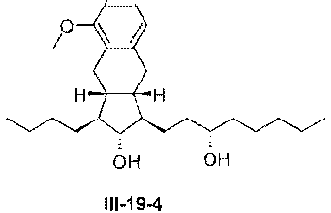 | ¹H NMR (400 MHz, d⁶-DMSO)δ7.06(dd, J =8.0 Hz, J=7.6 Hz,1H),6.80(d, J=7.6 Hz,1H),6.73(d,7=7,2 Hz,1H),4.38(d, J=6.8 Hz,1H),4.21(d, J=5.2 Hz,1H),3.74(s,3H),3.37-3.32(m,1H),3.05-2.97(m,1H),2.67-2.58(m,2H),2.49-2.43(m,1H),2.36(dd, J=13.2 Hz, J=4.8Hz,1H),1.80-1.68(m,2H),1.65-1.56(m,1H),1.54-1.16(m,17H),1.09-0.99(m,2H),0.89(1, J=7.2 Hz,3H),0.87(t, J=6.8 Hz,3H). |
| | LRMS: Calcd. for C₂₆H₄₂O₃[M + Na]⁺:425.3, found 425.3. |
| 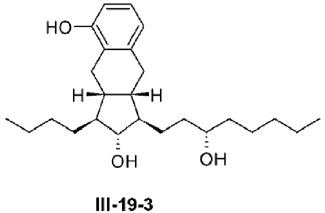 | ¹H NMR (400 MHz, d⁶-MSO)δ9,04(s,1H),6.87(t,7=7.6 Hz,1H),6.63(d,7=8.0 Hz,1H),6.56(d,/=7.2 Hz,1H),4.39(d, J =6.8 Hz,1H),4.22(t,7=5.2 Hz,1H),3.38-3.30(m,1H),3.03-2.96(m,1H),2.67-2.57(m,2H),2.40(dd, J=14.4 Hz, J=4.8 Hz,1H),2.31(dd, J=14.4Hz, J=4.8Hz,1H),1.77-1.56(m,3H),1.53-1.16(m,15H),1.13-1.00(m,4H),0.89(t,7=7.2 Hz,3H),0.86(t, J=6.8 Hz,3H). |
| | LRMS: Calcd. for C₂₅H₄₀O₃[M + Na]⁺:411.3, found 411.3. |
| 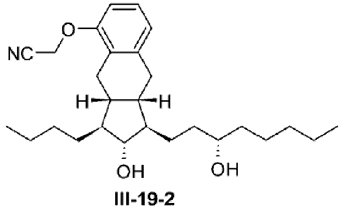 | ¹H NMR (400 MHz, d⁶-DMSO)δ7.14(t, J =8.0 Hz, IH),6.95(d, J=8.4Hz, IH),6.87(d,7=7.2 Hz, IH),5.13(s,2H),4.41(d, J=6.4 Hz, IH),4.21(d,7=5.6 Hz, IH),3.38-3.32(m, IH),3.06-2.97(m, IH),2.70-2.60(m, IH),2.54-2.36(m,2H),1.83-1.72(m,2H),1.67-1.15(m,19H),1.08-0.99(m,2H),0.88(t, J=6.8 Hz,3H),0.87(t, J =6.8 Hz,3H). |
| | LRMS: Calcd. for C₂₇H₄₁NO₃[M + Na]⁺:450.3, found 450.2. |
| 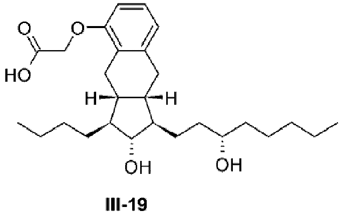 | ¹H NMR (400 MHz, d⁶-DMSO)δ6.95(t, J =7.6 Hz, IH),6.66(d, J=7.6Hz, IH),6.59(d,7=8.4 Hz, IH),4.52-4.13(m,3H)4.22(s,2H),),3.39-3.32(m, IH),3.03(t, J=9.6 Hz, IH),2.83-2.74(m, IH),2.68-2.62(m, IH),2.43-2.28(m,2H),1.74-1.56(m,3H),1.54-1.19(m,17H),1.17-1.09(m,2H),0.94(t,7=7.6 Hz,3H),0.87(t,J=6.4 Hz,3H) LRMS: Calcd. for C₂₇H₄₂O₅[M + Na]⁺:469.3, found 469.3. |
| 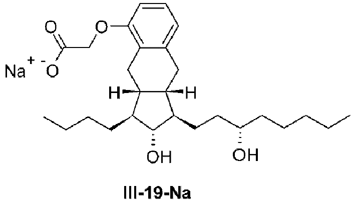 | ¹H NMR (400 MHz, D₂O)δ7.08(t, J=7.6 Hz,1H),6.78-6.89(m,2H),4.41(s,2H),3.52(s,1H),3.16(1,7=9.2 Hz,1H),2.82(d, J=13.6 Hz,1H),2.66(d, J=13.6 Hz,1H),2.51(d, J=14.0 Hz,1H),2.38(d, J =14.0 Hz,1H),1.83-1.63(m,3H),1.62-1.17(m,19H),0.99-0.85(m,6H). |
| | LRMS: Calcd. for C₂₇H₄₁NaO₅[M + H]⁺:469.3, found 469.3. |
| 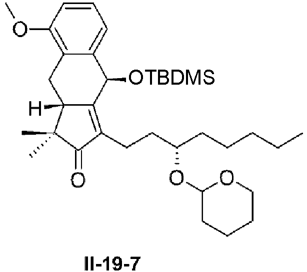 | ¹H NMR (400 MHz, d⁶-DMSO)δ7.30-7.25(m,1H),6.94-6.91(m,2H),5.59(d, J=8.0 Hz,1H),4.49-4.36(m,lH),3.78(s,3H),3.72-3.66(m,lH),3.36-3.33(m,1H),3.32(s,3H),3.30-3.21(m,1H),2.90-2.84(m,1H),2.33-2.05(m,3H),1.73-1.68(m,1H),1.61-1.28(m,9H),1.24-1.11(m,4H),1.06(s,3H),1.02(s,3H),0.84-0.77(m,12H),0.14-0.08(m,6H). |
| | LCMS: Calculated for C₃₅H₅₆O₅Si[M + Na]+:607.3, found 607.3. |
| 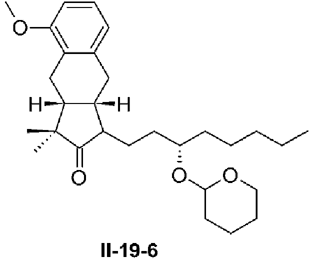 | ¹H NMR (400 MHz, d⁶-DMSO)δ7.11-7.07(m, IH),6.78-6.74(m,2H),4.60-4.57(m, IH),3.80-3.76(m,4H),3.56-3.49(m, IH),3.42-3.36(m, IH),3.09-3.03(m, IH),2.92-2.86(m, IH),2.68-2.60(m, IH),2.40-2.36(m,lH),2.12-1.94(m,3H),1.71-1.35(m,11H),1.26-1.24(m,7H),1.02(s,6H),0.87-0.84(m,3H). |
| | LCMS: Calculated for C₂₉H₄₄O₄[M + Na]⁺:479.3, found 479.3. |
| 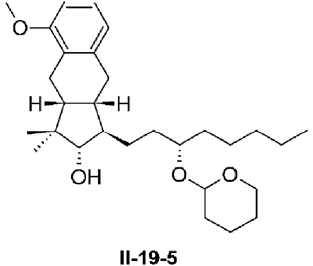 | ¹H NMR (400 MHz, d⁶-DMSO)δ7.05-7.02(t, J=8.0 Hz, IH),6.78(d, J=8.4 Hz, IH),6.72-6.69(m, IH),4.65-4.61(m, IH),4.57-4.54(m, IH),3.84-3.78(m, IH),3.75(s,3H),3.56-3.53(m, IH),3.44-3.40(m, IH),3.12-3.08(m, IH),2.91-2.86(m, IH),2.75-2.69(m, IH),2.31-2.23(m, IH),2.19-2.13(m, IH),1.73-1.54(m,6H),1.53-1.36(m,8H),1.35-1.20(m,7H),0.95(s,3H),0.88-0.84(m,3H),0.69(s,3H). |
| | LCMS: Calculated for C₂₉H₄₆O₄[M + Na]+:481.4, found 481.4. |
| 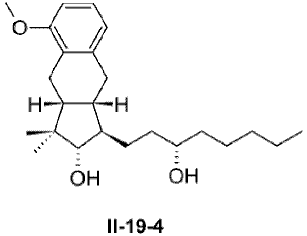 | ¹H NMR (400 MHz, d⁶-DMSO)δ7.05-7.01(t, J=8.0 Hz,1H),6.78(d, J=8.0 Hz,1H),6.71(d,7=8.0 Hz,1H),4.54(d, J=6.8Hz,1H),4.23(d,7=5.2 Hz,1H),3.75(s,3H),3.37(s,1H),3.11-3.08(m,1H),2.92-2.86(m,1H),2.74-2.69(m,1H),2.29-2.23(m,1H),2.19-2.13(m,1H),1.74-1.63(m,1H),1.53-1.46(m,2H),1.43-1.36(m,4H),1.29-1.26(m,8H),0.96(s,3H),0.88-0.85(m,3H),0.70(s,3H). |
| | LCMS: Calculated for C₂₄H₃₃O₃[M + Na]+:397.3, found 397.3. |
| 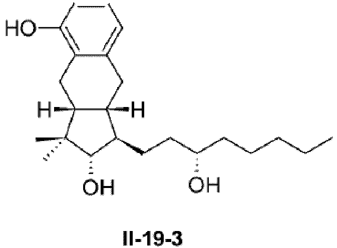 | ¹H NMR (400 MHz, d⁶-DMSO)δ59.06(s,1H),6.86-6.83(t, J=8.0 Hz,1H),6.62-6,61(d, J=8.0 Hz,1H),6.54(d, J=8.0 Hz,1H),4.52(d,7=6.8 Hz,1H),4.23(d,J=5.2 Hz,1H),3.37(s,1H),3.12-3.08(m,1H),2.87-2.82(m,1H),2.69-2.64(m,1H),2.26-2.20(m,1H),2.17-2.10 1.70-1.63(m, 2H),1.53-1.44(m,2H),1.41-1.35(m,4H),1.29-1.21(m,7H),0.96(s,3H),0.88-0.85(m,3H),0.70(s,3H). |
| | LCMS: Calculated for C₂₃H₃₆O₃[M + Na]+:383.3, found 383.3. |
| 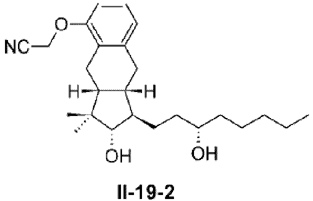 | The product II-19-2 crude was used in the next step directly. |
| | LC-MS characterization confirmed it as the target product. |
| | LCMS: Calculated for C₂₆H₄₀O₅[M + Na]+:455.3, found 455.3. |
| 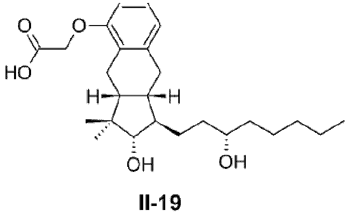 | ¹H NMR (400 MHz, d⁶-DMSO)δ12.82(s,lH),7.02-6.98(t, J=8.0 Hz,1H),6.73(d, J=8.0 Hz,1H),6.65(d, J=8.0 Hz,1H),4.64(s,1H),4.56(d,7=6.8 Hz,1H),4.23(d,7=5.2 Hz,1H),3.37(s,1H),3.13-3.08(m,1H),3.18-2.96(m,1H),2.76-2.71(m,1H),2.29-2.24(m,1H),2.21-2.15(m,1H),1.74-1.65(m,2H),1.54-1.46(m,2H),1.45-1.36(m,4H),1.31-1.21(m,8H),0.97(s,3H),0.88-0.85(m,3H),0.73(s,3H). LCMS: Calculated for C₂₅H₃₈O₅[M + Na]+:441.3, found 441.3. |
| 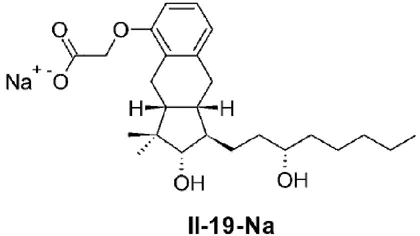 | ¹H NMR (400 MHz, D₂O)δ7.05-7.01(t, J =8.0 Hz,1H),6.75(d, J=8.0 Hz,1H)6.65(d,7=8.0 Hz,1H),4.37(s,2H),3.56(d, J =6.8 Hz,1H),3.22(d, J=5.2 Hz,1H),2.99-2.93(m,1H),2.73-2.68(m,1H),2.29-2.20(m,2H),1.73-1.69(m,1H),1.64-1.55(m,2H),1.49-1.38(m,5H),1.29-1.16(m,7H),0.96(s,3H),0.80-0.78(m,3H),0.69(s,3H). |
| | LCMS: Calculated for C₂₅H₃₇NaO₅[M + H]+:441.3, found 441.3. |

III-20-6 was prepared as per the following procedure, while the remaining intermediates, III-20 and III-20-Na was prepared according to the aforementioned Steps 3-8.

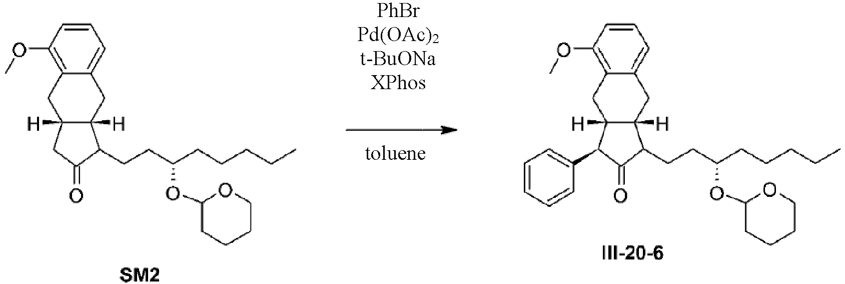

In a reaction flask, starting material (SM2), PhBr (1.5 equivalents), sodium tert-butoxide (1.5 equivalents), catalytic amounts of Pd(OAc)₂ and XPhos and the solvent toluene were added. The mixture was heated to 80°C and stirred. After the starting material was consumed, the reaction was quenched with saturated NaCl aqueous solution, and the mixture was extracted, dried, filtered, concentrated, and purified by column chromatography to obtain product III-20-6. The product was characterized by LC-MS and ¹H-NMR, respectively.

**Table B**

| Compound structure and number | Characterization data |
|---|---|
| 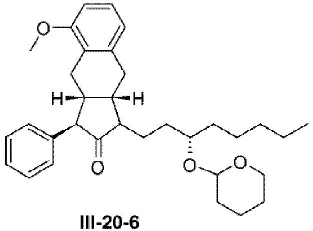 | ¹HNMR (400 MHz, CDCI₃)δ7.35-7.32(m,2H),7.27-7.24(m,1H),7.17-7.08(m,3H),6.81-6.69(m,2H),4.70-4.62(m,1H),3.95-3.90(m,1H),3.81-3.78(m,3H),3.70-3.63(m,1H),3.52-3.48(m,1H),2.86-2.75(m,3H),2.70-2.63(m,1H),2.44-2.38(m,1H),1.75-1.45(m,12H),1.33-1.26(m,9H),0.89-0.87(m,3H). |
| | LCMS: Calculated for C₃₃H₄₄O₄[M + Na]+:527.3, found 527.3. |
| 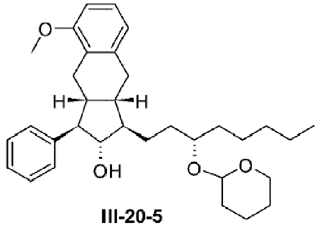 | The product III-20-5 crude was used in the next step directly. |
| | LC-MS characterization confirmed it as the target product. |
| | LCMS: Calculated for C₃₃H₄₆O₄[M + Na]+:529.3, found 529.3. |
| 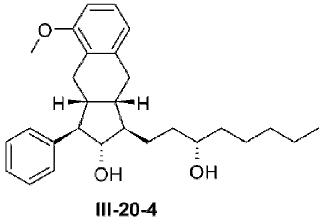 | ¹H NMR (400 MHz, d⁶-DMSO)δ7.33-7.29(m,2H),7.22-7.18(m,3H),7.12-7.08(t, J=8.0 Hz,1H),6.83(d, J=8.0 Hz,1H),6.78(d,7=7.2 Hz,1H),4.49(d,J=6.8 Hz,1H),4.25(d,7=5.6 Hz,1H),3.73(s,3H),3.59-3.58(m,1H),3.38(m,1H),2.73-2.68(m,1H),2.47-2.41(m,3H),2.18-2.15(m,2H),1.73-1.71(m,1H),1.52-1.17(m,13H),0.88-0.85(m,3H). |
| | LCMS: Calculated for C₂₈H₃₈O₃[M + Na]+:445.3, found 445.3. |
| 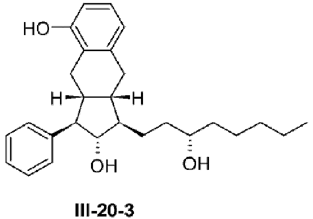 | ¹H NMR (400 MHz,(d⁶-DMSO)δ9.09(s,1H),7.32-7.28(m,2H),7.24-7.18(m,3H),6.93-6.87(m,1H),6.67-6.62(m,2H),4.48(d, J=6.8 Hz,1H),4.26(d, J=5.2 Hz,1H),3.60-3.58(m,1H),3.38(m,1H),2.66-2.63(m,1H),2.43-2.39(m,3H),2.19-2.14(m,2H),1.78-1.67(m,1H),1.51-1.36(m,4H),1.31-1.19(m,9H),0.88-0.84(m,3H). |
| | LCMS: Calculated for C₂₇H₃₆O₃[M + Na]+:431.2, found 431.2. |
| 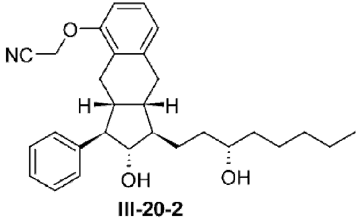 | ¹H NMR (400 MHz, d⁶-DMSO)δ7.32-7.28(m,2H),7.24-7.16(m,4H),6.98(d, J=8.0 Hz,1H),6.93-6.93(d,7=8.0 Hz,1H),5.14(s,1H),4.51-4.49(d, J=6.8Hz,1H),4.25(d,7=5,6 Hz,1H),3.63-3.56(m,1H),3.38(m,1H),2.74-2.69(m,1H),2.47-2.38(m,2H),2.24-2.15(m,2H),2.04-1.98(m,1H),1.76-1.70(m,1H),1.55-1.17(m,14H),0.90-0.84(m,3H). |
| | LCMS: Calculated for C₂₀H₃₇NO₃[M + Na]+:470.3, found 470.3. |
| 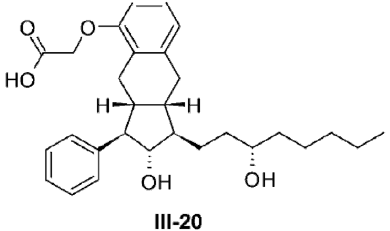 | ¹H NMR (400 MHz, d⁶-DMSO)δ12.91(m,1H),7.31-7.25(m,4H),7.21-7.17(m,1H),7.06(d,7=8.0 Hz,1H),6.80(d, J=7.6Hz,1H),6.71-6.68(d, J=8.4 Hz,1H),4.63(s,2H),4.50(d,7=6.8 Hz,1H),4.27(d, J =4.8 Hz,1H),3.63-3.59(m,2H),2.72-2.67(m,1H),2.20-2.14(m,2H),2.01-1.99(m,1H),1.78-1.72(m,2H),1.54-1.17(m,14H),0.89-0.85(m,3H). |
| | LCMS: Calculated for C₂₉H₃₈O₅[M + Na]+:490.2, found 490.2. |
| 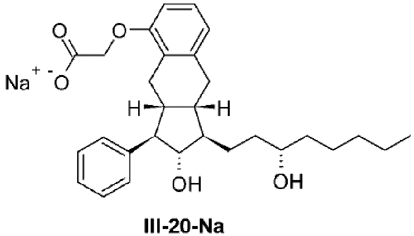 | ¹H NMR (400 MHz, D₂O)δ7.33-7.30(m,2H),7.23-7.21(m,3H),7.10(d,7=8.0 Hz,1H),6.83(d,J=7.2 Hz,1H),6.69(d, J=8.4 Hz,1H),4.39-4.31(m,2H),3.76-3.71(m,1H),3.61-3.55(m,1H),2.67-2.65(m,1H),2.53-2.43(m,3H),2.26-2.19(m,1H),2.09-2.05(m,1H),1.72-1.17(m,14H),0.80-0.77(m,3H). |
| | LCMS: Calculated for C₂₉H₃₇NaO₅[M + H]+:489.3, found 489.3. |

In Step 5, products III-21-3 and III-21'-3 were obtained respectively, and the two products were further processed according to Steps 6-8 to obtain III-21 and III-21' and their corresponding sodium salts III-21-Na and III-21'-Na.

**Table C**

| Compound structure and number | Characterization data |
|---|---|
| 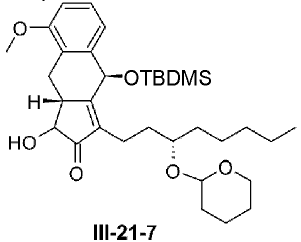 | ¹H NMR (400 MHz, d⁶-DMSO)δ7.29-7.24(m,1H),6.99-6.93(m,2H), 5.84-5.81(t, J=5.6 Hz,1H),5.65(d, J=12 Hz,1H),4.56-4.44(m,1H),3.81(s,3H),3.77-3.71(m,2H),3.46-3.39(m,1H),3.31-3.23(m,1H),3.02-3.01(m,1H),2.75-2.66(m,1H),2.35-2.16(m,1H),1.75-1.56(m,2H),1.49-1.44(m,8H),1.28-1.12(m,8H),0.88-0.77(m,12H),0.14-0.02(m,6H). |
| | LCMS: Calculated for C₃₃H₅₂O₆Si[M + Na]+:595.2,found 595.2. |
| 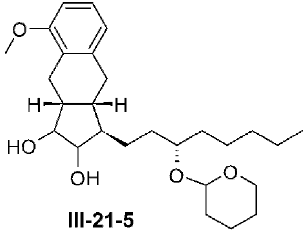 | ¹H NMR (400 MHz, d⁶-DMSO)δ7.08-7.05(m,1H),6.82-6.79(m,1H),6.73-6.68(m,1H),4.67-4.61(m,2H),4.55-4.52(m,1H),3.82-3.78(m,1H),3.54-3.40(m,3H),3.18-3.08(m,1H),2.96-2.80(m,1H),2.66-2.57(m,2H),2.35-2.31(m,1H),1.73-1.71(m,1H),1.65-1.17(m,23H),0.87-0.85(m,3H). LCMS: Calculated for C₂₇H₄₂O₅[M + Na]+:469.2, found 469.2. |
| 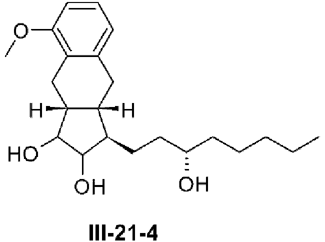 | The product III-20-4 was used in the next step directly. LC-MS characterization confirmed it as the target product. |
| | LCMS: Calculated for C₂₂H₃₄O₄[M + Na]+:385.2, found 385.2. |
| 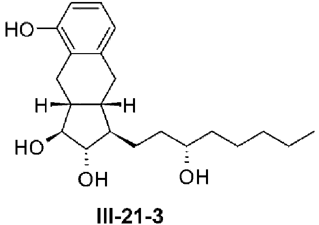 | ¹H NMR (400 MHz, d⁶-DMSO)δ9.05(s,1H),6.87(t,7=8.0 Hz,1H),6.64(d, J=8.0 Hz,1H),6.57-6.55(d, J=7.2 Hz,1H),4.63(d, J=5.2 Hz,1H),4.51(d, J=5.6 Hz,1H),4.22-4.20(d, J=5.6Hz,1H),3.20-3.14(m,1H),3.01-2.95(m,1H),2.61-2.53(m,3H),2.33-2.28(m,1H),1.90-1.82(m,2H),1.61-1.57(m,1H),1.45-1.25(m,12H),1.02-0.98(m,1H),0.88-0.85(m,3H). |
| | LCMS: Calculated for C₂₁H₃₂O₄[M + Na]+:371.3, found 371.3. |
| 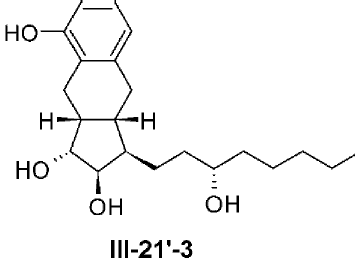 | ¹H NMR (400 MHz, d⁶-DMSO)δ9.11(s,1H),6.86(3 J=8.0 Hz,1H),6.57(d, J =7.6 Hz,1H),6.52(d,7=7.6 Hz,1H),4.81(d, J=5.2 Hz,1H),4.44(d, J=5.6 Hz,1H),4.20(d, J=5.2Hz,1H),3.81-3.77(m,1H),3.52-3.48(m,1H),3.39-3.34(m,1H),3.09-3.03(m,1H),2.88-2.83(m,1H),2.40-2.33(m,1H),2.09-2.02(m,1H),1.56-1.26(m,15H),0.87(t, J=6.8 Hz,3H). |
| | LCMS: Calculated for C₂₁H₃₂O₄[M + Na]+:371.3, found 371.3. |
| 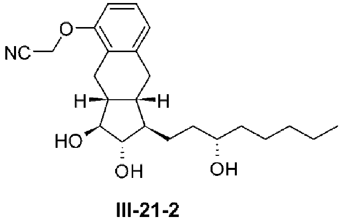 | ¹H NMR (400 MHz, d⁶-DMSO)δ7.14(t, J =8.0 Hz, IH),6.95(d,7=8.4 Hz, IH),6.88(d,7=7.2 Hz, IH),5.13(s,2H),4.71(d, J=5.2 Hz, IH),4.56(d, J=5.6 Hz, IH),4.23(d, J=5.2 Hz, IH),3.21-3.15(m, IH),2.99-2.94(m, IH),2.65-2.61(m,3H),2.40-2.36(m, IH),1.94-1.85(m,2H),1.64-1.57(m,1H),1.43-1.25(m,12H),1.02-0.98(m,1H),0.88-0.85(m,3H). |
| | LCMS: Calculated for C₂₃H₃₃NO₄[M + Na]+:410.3, found 410.3. |
| 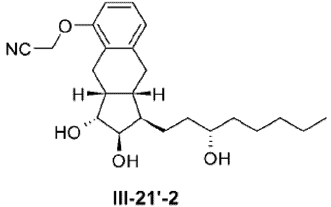 | ¹H NMR (400 MHz, d⁶-DMSO)δ7,12(t, J =8.0 Hz,1H),6.87(d,7=7.6 Hz,1H),6.82(d, J=7.6 Hz,1H),5.15(s,2H),4.88(d,7=4.4 Hz,1H),4.48(d, J=5.6 Hz,1H),4.20(d,7=4.4 Hz,1H),3.80(s,1H),3.52(s,1H),3.12-3.07(m,1H),2.95-2.91(m,1H),2.16-2.09(m,1H),1.53-1.25(m,17H),0.87-0.85(m,3H). |
| | LCMS: Calculated for C₂₃H₃₃NO₄[M + Na]+:410.3, found 410.3. |
| 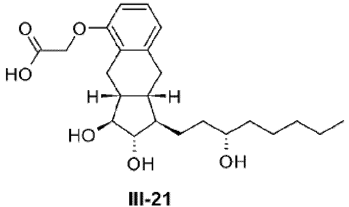 | NMR (400 MHz, d⁶-DMSO)δ12.97(s,1H),7.03(t, J=8.0 Hz,1H),6.75(d, J =7.2 Hz,1H),6.68(d, J=8.4 Hz,1H),4.78(s,1H),4.63(s,2H),4.58(d,7=4.8 Hz,1H),4.24(d,7=5.2 Hz,1H),3.22-3.16(m,1H),3.06-3.02(m,1H),2.75-2.70(m,1H),2.65-2.57(m,2H),2.36-2.31(m,1H),1.90-1.81(m,2H),1.64-1.58(m,1H),1.47-1.25(m,12H),1.06-1.02(m,1H),0.88-0.85(m,3H). |
| | LCMS: Calculated for C₂₃H₃₄O₆[M + Na]+:429.2, found 429.2. |
| 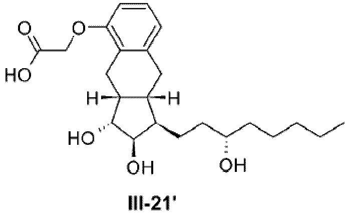 | ¹H NMR (400 MHz, d⁶-DMSO)δ12.96(s,1H),7.01(t, J=8.0 Hz,1H),6.70(d, J =7.6 Hz,1H),6.61(d,7=8.0 Hz,1H),4.89(d,7=5.2 Hz,1H),4.65(s,2H),4.48(d, J=6.0 Hz,1H),4.22(d, J=5.2 Hz,1H),3.80-3.79(m,1H),3.54-3.50(m,1H),3.18(m,1H),2.92-2.87(m,1H),2.44-2.33(m,1H),2.16-2.01(m,1H),1.56-1.26(m,16H),0.88-0.85(m,3H). |
| | LCMS: Calculated for C₂₃H₃₄O₆[M + Na]+:429.3, found 429.3 o |
| 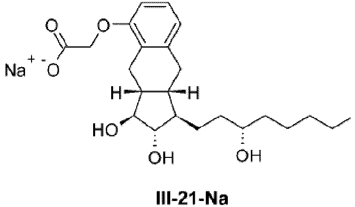 | ¹H NMR (400 MHz, D₂O)67.09(t, J=8.0 Hz,1H),6.81(d,7=7.2 Hz,1H),6.69(d,7=8.0 Hz,1H),4.38(s,2H),3.57(m,1H),3.40-3.45(m,1H),3.05-3.01(m,1H),2.89-2.84(m,1H),2.61-2.40(m,3H),2.09-2.08(m,2H),1.59-1.51(m,2H),1.47-1.31(m,5H),1.27-1.20(m,5H),1.01(m,1H),0.79-0.76(m,3H). |
| | LCMS: Calculated for C₂₃H₃₃NaO₆[M + H]+:429.1, found 429.1. |
| 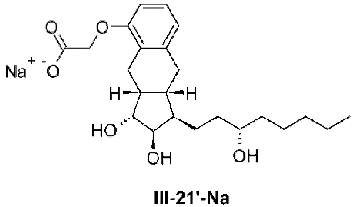 | ¹H NMR (400 MHz, D₂O)δ7.06(t, J=8.0 Hz, 1H),6.75(d,7=7.6 Hz,1H),6.57(d,7=8.0 Hz,1H),4.39(s,2H),4.03-4.00(m,1H),3.72-3.69(m,1H),3.62-3.61(m,1H),3.20-3.17(m,1H),2.98-2.95(m,1H),2.50-2.43(m,1H),2.28-2.21(m,1H),1.67(m,1H),1.51-1.29(m ,9H),1.27-1.21(m,5H),0.79-0.76(m,3H). |
| | LCMS: Calculated for C₂₃H₃₃NaO₆[M + H]+:429.1, found 429.1. |

### Performance Testing and Results

### Purchase and acclimatization

Twenty-eight 7-week-old male Sprague-Dawley rats (average weight of 200-250 g) were purchased from Vital River and were raised at the experimental site for 1 week of acclimatization (temperature 20°C-26°C, humidity 30%-70%, light-dark cycle ratio 1:1) until they were 8 weeks old.

### Grouping

Twenty-eight rats were randomized into the following 6 groups, including dosing groups, positive control groups, a negative control group and a healthy control group, and each was marked with ear tags.

| Modeling | Group | Drug for intragastric administration | Dose (mg/kg/day) | Number of animals |
|---|---|---|---|---|
| MCT modeling | Group 1 | Compound 1H | 6 | 4 |
| | Group 2 | Compound | 3 | 4 |
| | Group 3 | Compound C1H | 6 | 5 |
| | Group 4 | Compound C1M | 3 | 5 |
| | Group 5 | Normal saline | / | 5 |
| Normal saline | Group 6 | Normal saline | / | 5 |
| Total | | | | 28 |

| | | | | |
|---|---|---|---|---|
| Note 1: Group 1 and Group 2 were treated with treprostinil-like test compound (Compound 1 is Compound III-2-Na), Group 3 and Group 4 were the positive control groups for treprostinil (Compound C1 is Treprostinil sodium 5.5-hydrate), Group 5 was the negative control group, and Group 6 was the healthy control group. Note 2: The suffix H represents the high-dose group (6 mg/kg); M represents the medium-dose group. | | | | |

### 3. Modeling

(1) Preparation of monocrotaline (MCT) solution: An adequate amount of MCT was weighed into a 50 mL solution of 20% ethanol-normal saline. After gentle shaking, the solution was transferred to a 50-mL centrifuge tube, and placed tilted in a thermostatic shaker at 37°C, 220 rpm for 12-14 hours to fully dissolve until no visible crystalline particles observed.
(2) Injection of MCT solution: The skin on the dorsal cervical region of the rats was gently lifted, MCT solution was subcutaneously injected at a dose of 60 mg/kg using a 1-mL syringe and the day was recorded as Day 0.
(3) Healthy control: The same volume of normal saline was injected in the same manner.

### 4. Preparation of test compound

(1) Compound 1 and Compound C1 can be directly dissolved in water. According to the concentration of 0.6 mg/mL and 1.2 mg/mL (3-mg/kg/day and 6-mg/kg/day dose groups), appropriate amounts of the test compound were weighed and dissolved in corresponding volume of pure water by shaking to obtain transparent solution, respectively;
(2) The resulting solutions were stored in a 4°C refrigerator. Remove and equilibrate to room temperature before use;

### 5. Administration

Starting from Day 14, the rats were weighed daily, and the corresponding solutions (test compound or normal saline) were administered intragastrically according to the grouping protocol until Day 27, for a total of 14 days; the intragastric dose was 0.5 mL/100 g, and the body weight and survival data of the rats were recorded.

### 6. Hemodynamic measurements

On Day 28, the hemodynamic status of the rats was assessed using right cardiac catheterization:
(1) The PE-50 catheter, pressure transducer and pressure measuring workstation were prepared; the tubing was rinsed with heparinized saline to ensure that no bubbles remained in the tubing; the catheter was corrected to zero;
(2) The rats were anesthetized with 2% isoflurane at a flow rate of 2 L/min, and their limbs and incisors were fixed on the operating table with the cranial end to the operator;
(3) The fur on the surgical area of cervical region was shaved;
(4) The cervical skin was cut along the midline, pulled and fixed using hemostats, and the tissue was separated layer by layer using scissors to expose the right internal jugular vein;
(5) The internal jugular vein was bluntly dissected and isolated using hemostats;
(6) The distal end of the internal jugular vein was ligated using 4-0 sutures, and a suture was passed through the proximal end for later use;
(7) A V-shaped incision was made in the internal jugular vein using ophthalmic scissors, and a PE-50 catheter was inserted through the incision. After the catheter was placed, the pre-placed suture at the proximal end of the internal jugular vein was ligated to secure the catheter;
(8) The catheter reached the right atrium and right ventricle in turn; the right atrial pressure and right ventricular pressure were recorded; at least 5 stable waveforms were recorded at each site; 5 continuous waveforms were taken as segments, and more than 2 segments were intercepted to calculate the right ventricular systolic pressure (RVSP);
(9) The PE-50 catheter was removed and the examination was completed.

### 7. Sampling

(1) After the hemodynamic evaluation was completed, the rats were anesthetized by intraperitoneal injection of 10% chloral hydrate at a dose of 0.5 mL/100 g, and the samples were collected after weighing;
(2) After opening the abdominal cavity, blood was drawn as much as possible using a 5-mL syringe from the inferior vena cava into a tube containing EDTA as anticoagulant. The tube was shaken upside down 6-8 times to mix;
(3) The lungs and heart were exposed by further opening the chest cavity;
(4) The left atrial appendage was cut open, a tube of heparinized saline was slowly injected using a 5-mL syringe into the right atrial appendage to flush the pulmonary vessels;
(5) The rat heart, lung, liver, spleen and kidney tissues were kept and frozen, and fixed with formaldehyde solution.

### 8. Fulton index measurement

After separating the heart, the non-ventricular parts (such as the atria, blood vessels, and valve rings) were cut off. The intraventricular thrombus was removed as much as possible using forceps. The right ventricle (RV) and left ventricle (LV) + ventricular septum (LV+S) were separated by cutting along tense junction between the RV and interventricular septum (S) using straight scissors. The tissues were dried with gauze and weighed separately. RV/(LV+S) was calculated as the Fulton index.

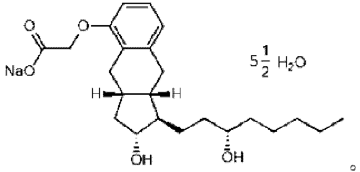

The test results are as follows:

### Body weight

1. Body weight on Day 0: The average body weights of Groups 1-6 on Day 0 were 347 ± 12.77, 315 ± 3.63, 316 ± 4.35, 330 ± 25.11, 315 ± 13.50, and 314 ± 5.35 g, respectively. The average body weight of Group 1 was significantly greater than that of Groups 2, 3, 5, and 6 (P < 0.01), and there was no significant difference among the groups.
2. Body weight on Day 14: The average body weights of Groups 1-6 on Day 14 were 440 ± 32.61, 428 ± 29.90, 413 ± 15.81, 430 ± 34.15, 406 ± 33.50, and 425 ± 20.48 g, respectively, indicating no significant difference among the groups.
3. Body weight at sampling: The body weights of Groups 1-6 at sampling were 436 ± 44.27, 440 ± 55.00, 436 ± 9.65, 431 ± 58.03, 406 ± 42.45, and 445 ± 25.06 g, respectively, indicating no significant difference among the groups.

Note: Due to the limited total amount of Compound 1, rats in Group 1 were actually administered for 13 days, rats in Group 2 were actually administered for 12 days, and all were measured and sampled on Day 27; rats in Groups 3-6 were actually administered for 14 days, and all were measured and sampled on Day 27.

**Table 1 Comparison of body weight changes of rats in each group**

| Group | Test compound | Dose (mg/kg/day) | Body weight on Day 0 (g) | Body weight on Day 14 (g) | Body weight at sampling (g) |
|---|---|---|---|---|---|
| 1 | Compound | 6 | 347±12.77 | 440±32.61 | 436±44.27 |
| 2 | Compound 1M | 3 | 315±3.63 | 428±29.90 | 440±55.00 |
| 3 | Compound C1H | 6 | 316+4.35 | 413+15.81 | 436+9.65 |
| 4 | Compound C1M | 3 | 330+25.11 | 430+34.15 | 431+58.03 |
| 5 | Negative control | / | 315+13.50 | 406+33.50 | 406+42.45 |
| 6 | Healthy control | / | 314+5.35 | 425+20.48 | 445+25.06 |

### Survival

Due to the limited total amount of Compound 1, rats in Group 1 were actually administered for 13 days, and rats in Group 2 were actually administered for 12 days. All were measured and sampled on Day 27.

Rats in Groups 3-6 were actually administered for **14** days, and all were measured and sampled on Day 27.

### Hemodynamic results

The RVSPs of Groups 1-6 were 22 ± 4.36, 41 ± 16.27, 38 ± 8.86, 46 ± 13.45, 63 ± 7.34, and 22 ± 2.41 mmHg, respectively. The RVSPs of modeling rats in Groups 2-4 were significantly higher than those in the healthy control group. The RVSP of rats in the group administered with the compound at 16 mg/kg had decreased to the level of the healthy control group (22 ± 4.36 vs 22 + 2.41 mmHg, P = 0.939).

**Table 2 Comparison of RVSP of rats in each group**

| Group | Test compound | Dose (mg/kg/day) | RVSP mmHg |
|---|---|---|---|
| 1 | Compound 1H | 6 | 22+4.36 |
| 2 | Compound 1M | 3 | 41±16.27 |
| 3 | Compound C1H | 6 | 38+8.86 |
| 4 | Compound C1M | 3 | 46+13.45 |
| 5 | Negative control | / | 63±7.34 |
| 6 | Healthy control | / | 22±2.41 |

As shown in Table 2, it can be concluded that:
1) Dosing group: The RVSP of the dosing group administered with Compound 1 at 6 mg/kg was significantly lower than that at 3 mg/kg (22 ± 4.36 vs 41 ± 16.27 mmHg, P = 0.01), indicating a difference between the dosing groups;
2) Positive control group: Compound C1, treprostinil sodium 5.5-hydrate, at doses of 3 and 6 mg/kg, can significantly reduce RVSP in rats, which was still higher than that in the healthy control group. And there was no significant difference in the effects between the two dosing groups (38 ± 8.86 vs 46 ± 13.45 mmHg, P = 0,201);
3) The RVSP of rats in the dosing group administered with Compound 1 at 6 mg/kg was lower than that administered with treprostinil sodium 5.5-hydrate at the same dose (22 ± 4.36 vs 38 ± 8.86 mmHg, P = 0.013), indicating its pulmonary vasodilation effect was better than that of treprostinil sodium 5.5-hydrate at the same dose.

### Fulton index results

The Fulton indexes of Groups 1-6 were 0.25 ± 0.27, 0.37 ± 0.12, 0.37 ± 0.07, 0.38 ± 0.10, 0.61 ± 0.080, and 0.29 ± 0.023, respectively. The Fulton index of the negative control group was significantly higher than that of the healthy control group. The Fulton index of the dosing groups was significantly lower than that of the negative control group, but there was no significant difference among the groups.

**Table 3 Comparison of Fulton index of rats in each group**

| Group | Test compound | Dose (mg/kg/day) | Fulton index |
|---|---|---|---|
| 1 | Compound 1H | 6 | 0.25±0.27 |
| 2 | Compound 1M | 3 | 0.37+0.12 |
| 3 | Compound C1H | 6 | 0.37+0.07 |
| 4 | Compound C1M | 3 | 0.38+0.10 |
| 5 | Negative control | / | 0.61+0.080 |
| 6 | Healthy control | / | 0.29+0.023 |

As shown in Table 3, it can be concluded that:
1) Dosing group: The Fulton index of the dosing group administered with Compound 1 at 6 mg/kg was lower than that at 3 mg/kg, but no significant difference was observed between the two dose groups (0.25 ± 0.27 vs 0.37 ± 0.12, P = 0.06);
2) Positive control group: Compound C1, treprostinil sodium 5.5-hydrate, at doses of 3 and 6 mg/kg, can significantly reduce the Fulton index of rat heart, which was still higher than that of the healthy control group. And there was no significant difference in the effects between the two dosing groups (0.37 ± 0.07 vs 0.38 ± 0.10, P = 0.921);
3) The Fulton index of rat heart in the dosing group administered with Compound 1 at 6 mg/kg was lower than that administered with treprostinil sodium 5.5-hydrate at the same dose (0.25 ± 0.27 vs 0.37 ± 0.07, P = 0.029); the Fulton index of rat heart in the dosing group administered with Compound 1 at 3 mg/kg was not significantly different from that administered with treprostinil sodium 5.5-hydrate at the same dose.

In addition, according to the same experimental method as described above, the corresponding test results for Ralinepag are shown in Tables 4 and 5.

**Table 4 Comparison of RVSP of rats in each group**

| Group | Test compound | Dose (mg/kg/day) | RVSP mmHg |
|---|---|---|---|
| 7 | Ralinepag | 60(30mpk bid) | 22 |

**Table 5 Comparison of Fulton index of rats in each group**

| Group | Test compound | Dose (mg/kg/day) | Fulton index |
|---|---|---|---|
| 7 | Ralinepag | 60(30mpk bid) | 0.35 |

All documents mentioned in the present invention are incorporated herein by reference as if each were individually cited. In addition, it should be understood that, upon reading the foregoing disclosure of the present invention, those skilled in the art may make various modifications or alterations to the invention, and such equivalent forms also fall within the scope defined by the appended claims of this application.

## Claims

1. A compound represented by formula (I), or a solvate thereof, or a pharmaceutically acceptable salt thereof, 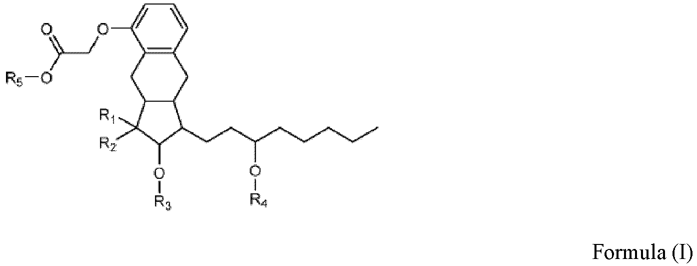 Wherein,
R₁ and R₂ are independently selected from the group consisting of hydrogen, deuterium, halogen, hydroxy, amino, substituted or unsubstituted C1-C6 alkyl, substituted or unsubstituted C2-C6 alkenyl, substituted or unsubstituted C2-C6 alkynyl, substituted or unsubstituted C1-C6 alkoxy, substituted or unsubstituted C3-C8 cycloalkyl, substituted or unsubstituted 4- to 8-membered heterocycloalkyl containing 1, 2 or 3 heteroatoms selected from N, O or S, substituted or unsubstituted C6-C10 aryl, substituted or unsubstituted 5- to 10-membered heteroaryl containing 1, 2 or 3 heteroatoms selected from N, O or S; 'substituted' refers to independent substitution by 1, 2 or 3 substituents selected from the group consisting of deuterium, halogen, hydroxyl, amino, C1-C6 alkyl, C2-C6 alkenyl, C2-C6 alkynyl, C1-C6 alkoxy, C3-C8 cycloalkyl, 4- to 8-membered heterocycloalkyl containing 1, 2 or 3 heteroatoms selected from N, O or S, R-substituted or unsubstituted C6-C10 aryl, R-substituted or unsubstituted 5-to 10-membered heteroaryl containing 1, 2 or 3 heteroatoms selected from N, O or S; R is selected from the group consisting of halogen, hydroxyl, C1-C6 alkyl, C1-C6 alkoxy;
or R₁, R₂ together with the C atoms to which they are attached form C3-C8 monocyclic cycloalkyl, C3-C8 bridged cycloalkyl, C7-C10 spirocycloalkyl, or 4- to 8-membered heterocycloalkyl containing 1, 2 or 3 heteroatoms selected from N, O or S;
and R₁ and R₂ are not simultaneously hydrogen;
R₃ and R₄ are independently selected from the group consisting of hydrogen, -(C=O)-C1-C6 alkyl, C1-C6 alkyl, halogenated C1-C6 alkyl, C3-C8 cycloalkyl, and halogenated C3-C8 cycloalkyl;
R₅ is selected from the group consisting of hydrogen, C1-C6 alkyl, halogenated C1-C6 alkyl, C3-C8 cycloalkyl, halogenated C3-C8 cycloalkyl, inorganic metal ion, and ammonium ion.

2. The compound according to claim 1, or a solvate thereof, or a pharmaceutically acceptable salt thereof, wherein R₁ is hydrogen;
R₂ is selected from the group consisting of halogen, C1-C6 alkyl, halogenated C1-C6 alkyl, C1-C6 alkyl substituted by R-substituted or unsubstituted C6-C10 aryl, hydroxy-substituted C1-C6 alkyl, C2-C6 alkenyl, C3-C8 cycloalkyl, 4- to 8-membered heterocycloalkyl containing 1, 2 or 3 heteroatoms selected from N, O or S, C6-C10 aryl, and 5-to 10-membered heteroaryl containing 1, 2 or 3 heteroatoms selected from N, O or S;
R is selected from the group consisting of halogen, C1-C6 alkyl, and C1-C6 alkoxy.

3. The compound according to claim 1, or a solvate thereof, or a pharmaceutically acceptable salt thereof, wherein R₁ is H, halogen, methyl, or ethyl;
R₂ is selected from the group consisting of halogen, methyl, ethyl, propyl, isopropyl, butyl, isobutyl, tert-butyl, neopentyl, tert-pentyl, trifluoromethyl, difluoromethyl, monofluoromethyl, monofluoroethyl, trifluoroethyl, difluoroisopropyl, hydroxy-substituted ethyl, vinyl, propenyl, ethynyl, propynyl, benzyl, diphenylmethyl, methoxybenzyl, monochlorobenzyl, phenyl-substituted ethyl, and hydroxy-substituted benzyl.

4. The compound according to claim 1, or a solvate thereof, or a pharmaceutically acceptable salt thereof, wherein R₃ and R₄ are hydrogen.

5. The compound according to claim 1, or a solvate thereof, or a pharmaceutically acceptable salt thereof, wherein the inorganic metal ion is selected from the group consisting of Li⁺, Na⁺, K⁺, Rb⁺, Cs⁺, Mg²⁺, Ca²⁺, Ba²⁺, Zn²⁺, and Al³⁺; and/or
The ammonium ion is an ammonium ion corresponding to a base selected from the group consisting of ammonia, methylamine, ethylamine, dimethylamine, trimethylamine, diethylamine, triethylamine, ethanolamine, diethanolamine, triethanolamine, tert-butylamine, tromethamine, meglumine, morpholine, piperazine, piperidine, pyridine, ethylenediamine, N,N'-dibenzylethylenediamine, proline, phenylalanine, aspartic acid, glutamic acid, and lysine.

6. The compound according to claim 1, or a solvate thereof, or a pharmaceutically acceptable salt thereof, wherein the compound has a structure represented by formula (II) or formula (III): 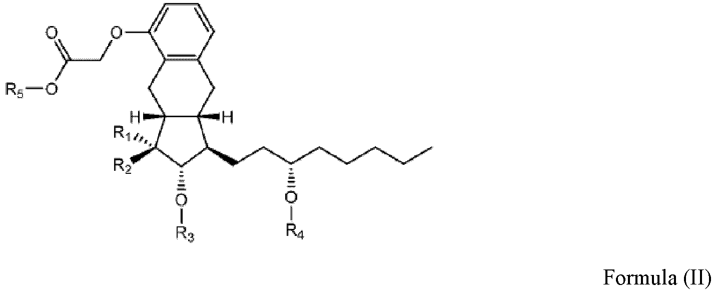 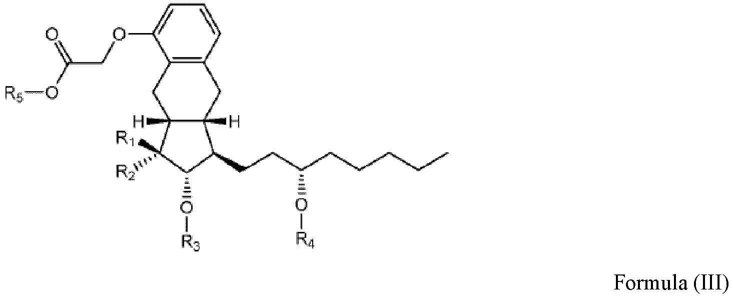 Wherein, R₁, R₂, R₃, R₄ and R₅ are as defined in claim 1.

7. The compound according to claim 1, or a solvate thereof, or a pharmaceutically acceptable salt thereof, wherein the compound is selected from the group consisting of the following:

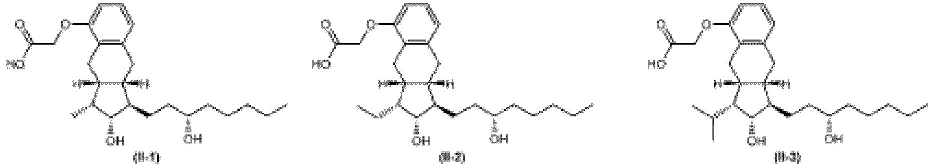

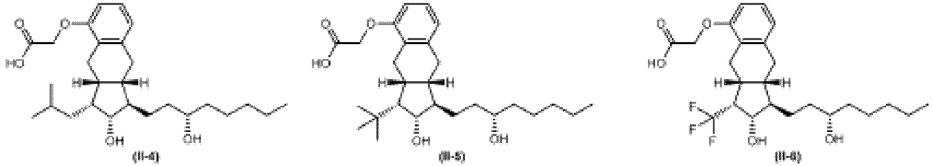

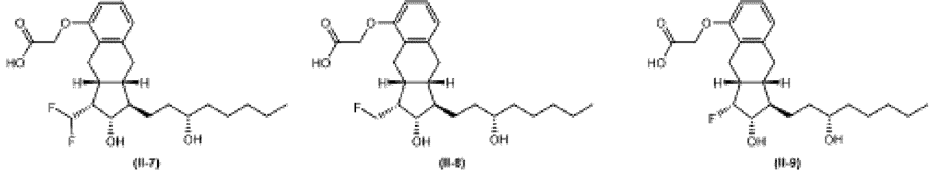

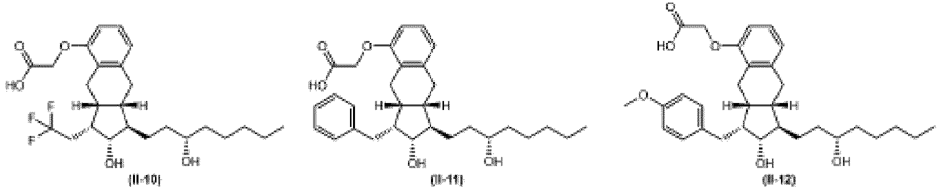

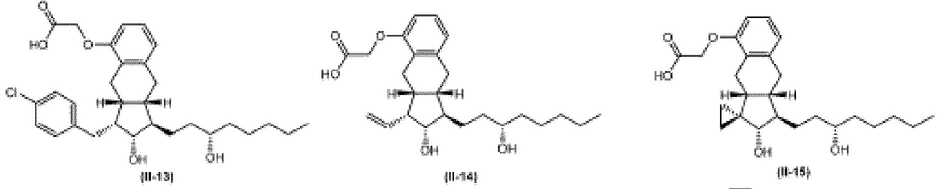

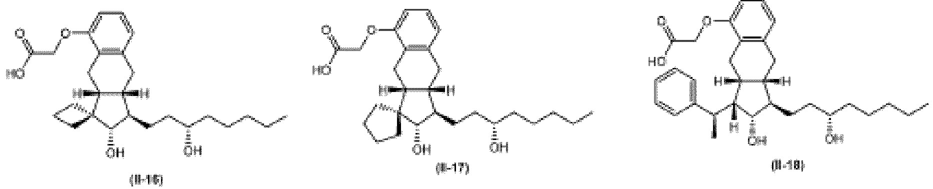

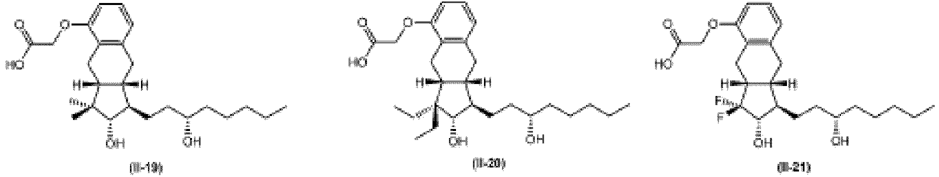

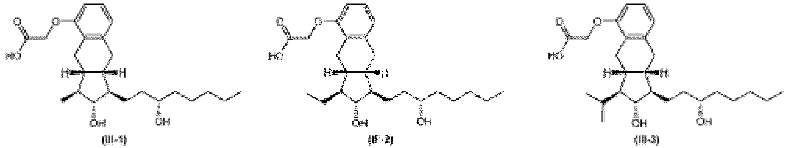

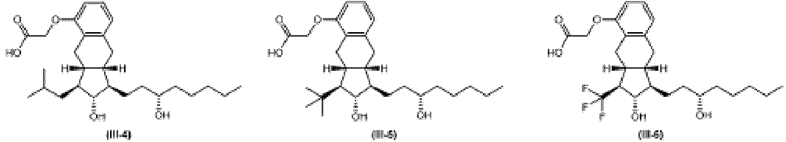

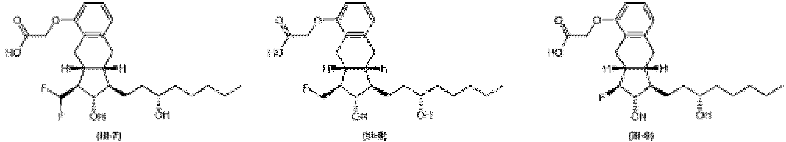

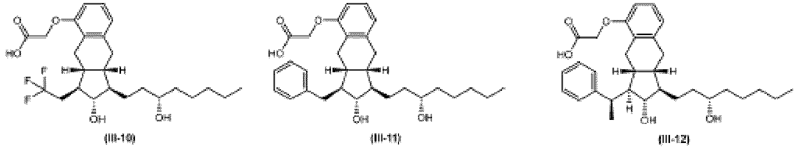

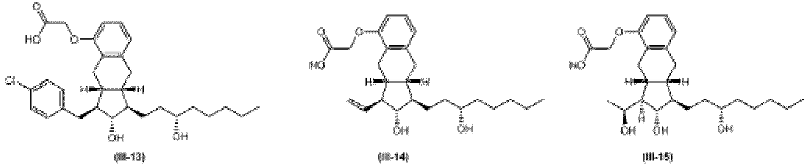

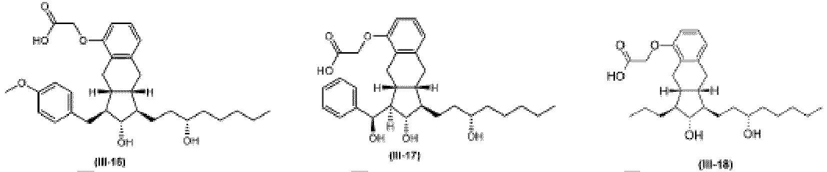

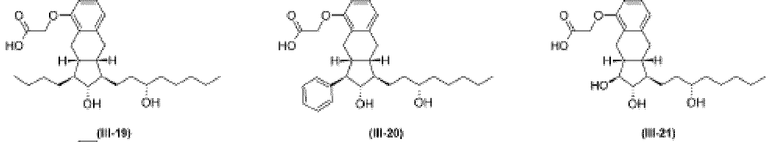

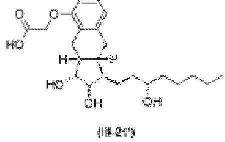

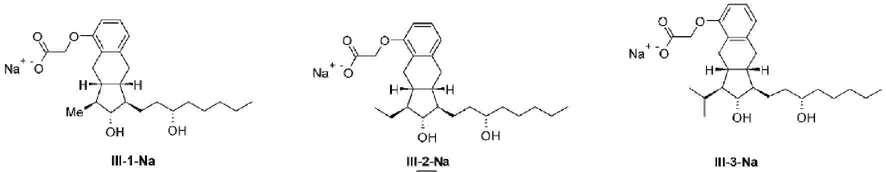

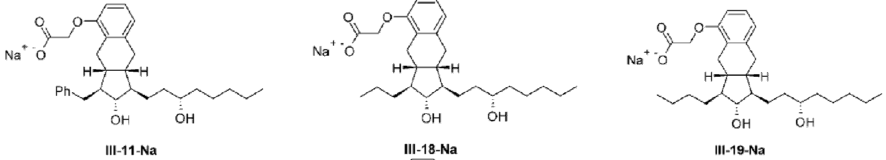

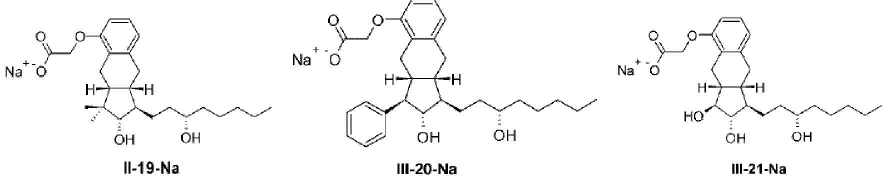

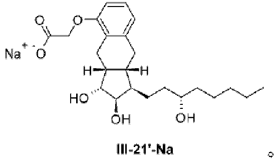

8. A pharmaceutical composition, wherein the composition comprises pharmaceutically acceptable carriers and a safe and effective amount of one or more compounds according to claim 1, or a solvate thereof, or a pharmaceutically acceptable salt thereof.

9. A use of the compound according to claim 1, or a solvate thereof, or a pharmaceutically acceptable salt thereof, in the manufacture of a medicament for treating pulmonary arterial hypertension.

10. The use of the compound according to claim 9, wherein the medicament is used for a purpose selected from the group consisting of the following:
1) Reducing right ventricular systolic pressure (RVSP);
2) Reducing Fulton index;
3) Reducing PVR;
4) Treating pulmonary hypertension caused by interstitial lung disease;
5) Treating pulmonary hypertension caused by COPD;
6) Treating idiopathic pulmonary fibrosis.
